(19) Europäisches Patentamt
European Patent Office
Office européen des brevets

(11) Veröffentlichungsnummer: **0 175 651**
**B1**

(12) **EUROPÄISCHE PATENTSCHRIFT**

(45) Veröffentlichungstag der Patentschrift: **12.04.89**

(51) Int. Cl.⁴: **C 07 D 239/42**, C 07 D 403/12, C 07 D 405/12, C 07 D 417/12, A 01 N 43/54

(21) Anmeldenummer: **85810421.9**

(22) Anmeldetag: **16.09.85**

(54) **Mikrobizide.**

(30) Priorität: **20.09.84 CH 4496/84**
**08.08.85 CH 3405/85**

(43) Veröffentlichungstag der Anmeldung:
**26.03.86 Patentblatt 86/13**

(45) Bekanntmachung des Hinweises auf die Patenterteilung:
**12.04.89 Patentblatt 89/15**

(84) Benannte Vertragsstaaten:
**AT BE CH DE FR IT LI LU NL SE**

(56) Entgegenhaltungen:
**EP-A- 0 129 433**
**DE-A- 1 926 547**

(73) Patentinhaber: **CIBA-GEIGY AG, Klybeckstrasse 141, CH-4002 Basel (CH)**

(72) Erfinder: **Zondler, Helmut, Dr., Oberwilerstrasse 49, CH-4103 Bottmingen (CH)**
Erfinder: **Eckhardt, Wolfgang, Dr., Breslauerstrasse 14, D-7850 Lörrach (DE)**
Erfinder: **Nyfeler, Robert, Dr., Bärenfelserstrasse 8, D-4057 Basel (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 175 651 B1

## Beschreibung

Die vorliegende Erfindung betrifft neue 5-(Arylmethylimino)-pyrimidine der nachstehenden Formel I. Die Erfindung betrifft ferner die Herstellung dieser Substanzen sowie Mittel, die als Wirkstoff mindestens eine dieser Verbindungen enthalten. Sie betrifft die Herstellung der Mittel sowie die Verwendung der Wirkstoffe oder der Mittel zur Bekämpfung schädlicher Mikroorganismen, vorzugsweise pflanzenschädigender Pilze und Bakterien. Die Erfindung betrifft überdies wichtige Zwischenprodukte der nachfolgend definierten Formeln II und VI.

Bei den erfindungsgemässen Verbindungen handelt es sich um solche der allgemeinen Formel I

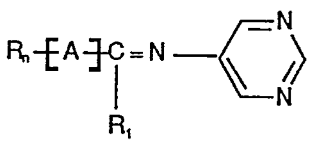

(I)

worin

A für Phenyl, Naphthyl, Biphenyl, Phenoxyphenyl oder Phenylthiophenyl steht;

R Halogen, Cyano, Nitro, $C_1$–$C_4$-Alkyl, $C_1$–$C_4$-Alkoxycarbonyl, Tris($C_1$–$C_4$-Alkoxy)silyl, Hydroxy, $C_1$–$C_4$-Alkoxy, $C_1$–$C_4$-Haloalkoxy, $C_1$–$C_4$-Haloalkyl oder Di($C_1$–$C_4$-alkyl)amino bedeutet;

n für 0, 1, 2, 3, 4 oder 5 steht;

$R_1$ eine der Gruppen $OR_2$, $SR_2$ oder $N(R_3)(R_4)$ repräsentiert, wobei $R_2$ für $C_1$–$C_8$-Alkyl, $C_3$-$C_8$-Alkenyl, $C_3$-$C_8$-Alkinyl oder für einen durch R einfach substituierten $C_1$–$C_8$-Alkylrest oder für einen unsubstituierten oder ein- bis dreifach durch R substituierten Rest aus der Reihe Phenyl, Phenalkyl($C_1$–$C_4$), $C_3$-$C_7$-Cycloalkyl oder Furfuryl steht; und

$R_3$ und $R_4$ unabhängig voneinander für Wasserstoff oder $C_1$–$C_4$-Alkyl stehen oder gemeinsam mit dem Aminstickstoff einen gesättigten fünf- oder sechs-gliedrigen Heterocyclus bilden, der als Heteroatom entweder nur den Aminstickstoff oder ein weiteres Heteroatom N, O oder S enthält.

Unter dem Begriff Alkyl selbst oder als Bestandteil eines anderen Substituenten wie Alkoxy, Haloalkyl, Haloalkoxy etc., sind je nach Zahl der angegebenen Kohlenstoffatome beispielsweise die folgenden geradkettigen oder verzweigten Gruppen zu verstehen: Methyl, Ethyl, Propyl, Butyl, Pentyl, Hexyl, Heptyl, Octyl usw. sowie ihre Isomeren, wie z. B. Isopropyl, Isobutyl, tert.-Butyl, Isopentyl usw. Halogen und Halo stehen stellvertretend für Fluor, Chlor, Brom oder Jod. Haloalkyl steht somit für einen einfach bis perhalogenierten Alkylrest, wie z. B. $CHCL_2$, $CH_2F$, $CCl_3$, $CH_2Cl$, $CHF_2$, $CHFCH_3$, $CH_2CH_2Br$, $C_2Cl_5$, $CH_2Br$, CHBrCl usw., vorzugsweise für $CF_3$. Alkenyl steht z. B. für Propenyl-(1), Allyl, Butenyl-(1), Butenyl-(2) oder Butenyl-(3) sowie Ketten mit mehreren Doppelbindungen. $C_3$-$C_7$-Cycloalkyl steht wahlweise für Cyclopropyl, Methylcyclopropyl, Cyclobutyl, Cyclopentyl, Cyclohexyl oder Cycloheptyl. Alkinyl bedeutet z. B. Propinyl-(2), Propargyl, Butinyl-(1), Butinyl-(2) usw. vorzugsweise Propargyl. Steht die Gruppe -$N(R_3)(R_4)$ für einen gesättigten fünf- oder sechsgliedrigen Heterocyclus mit N als Heteroatom, so sind als Heterocyclen bevorzugt: Pyrrolidin, Piperazin, Piperidin, Perhydrothiazin, Morpholin, Oxazolidin, Thiazolidin, Imidazolidin und Pyrazolin. Naphthyl repräsentiert α- und β-Naphthyl. Phenoxyphenyl steht für (o-Phenoxy)phenyl, (m-Phenoxy)phenyl und (p-Phenoxy)-phenyl. Phenylthiophenyl steht für (o-Phenylthio)phenyl, (m-Phenylthio)phenyl und (p-Phenylthio)phenyl. Ebenso kann im Biphenyl der zweite Phenylrest die ortho-, meta- oder para-Position einnehmen. Naphthyl, Phenoxyphenyl, Phenylthiophenyl und Biphenyl können in beiden Ringen substituiert sein.

Die Verbindungen der Formel I lassen sich auf dem Agrarsektor oder verwandten Gebieten präventiv und kurativ zur Bekämpfung phytopathogener Schädlinge, wie z. B. Pilzen, einsetzen. Die erfindungsgemässen Wirkstoffe der Formel I zeichnen sich in weiten Anwendungskonzentrationen durch eine hohe mikrobizide Aktivität, grosse Wirkungsbreite und geringe Phytotoxizität aus und können problemlos insbesondere im Agrarbereich eingesetzt werden.

Folgende Wirkstoffgruppen sind auf Grund ihrer ausgeprägten mikrobiziden, insbesondere pflanzenfungiziden Aktivität bevorzugt:

Gruppe I′: Verbindungen der Formel I, worin A für Phenyl, Naphthyl, Biphenyl, Phenoxyphenyl oder Phenylthiophenyl steht;

R Halogen, Cyano, Nitro, $C_1$–$C_4$-Alkyl, $C_1$–$C_4$-Alkoxy, $C_1$–$C_4$-Haloalkoxy, $C_1$–$C_4$-Haloalkyl oder Di($C_1$–$C_4$-alkyl)amino bedeutet;

n für 0, 1, 2, 3, 4 oder 5 steht;

$R_1$ eine der Gruppen $OR_2$, $SR_2$ oder $N(R_3)(R_4)$ repräsentiert, wobei

$R_2$ für $C_1$–$C_8$-Alkyl, $C_3$-$C_8$-Alkenyl, $C_3$-$C_8$-Alkinyl oder für einen unsubstituierten oder ein- bis dreifach durch R substituierten Rest aus der Reihe Phenyl, Benzyl, $C_3$-$C_7$-Cycloalkyl oder Furfuryl steht; und

$R_3$ und $R_4$ unabhängig voneinander für Wasserstoff oder $C_1$–$C_4$-Alkyl stehen oder gemeinsam mit dem Aminstickstoff einen gesättigten fünf- oder sechs-gliedrigen Heterocyclus bilden, der als Heteroatom entweder nur den Aminstickstoff oder ein weiteres Heteroatom enthält.

Gruppe I-A: Verbindungen der Formel I, worin A für Phenyl steht; R Hydroxy, Halogen, Cyano, Methyl, Ethyl, Methoxycarbonyl, Ethoxycarbonyl, Methoxy, Ethoxy, $CF_3$, Trimethoxysilyl, $C_1$–$C_2$-Haloalkoxy, $N(CH_3)_2$ oder $N(C_2H_5)_2$ bedeutet; n für 1, 2 oder 3 steht; $R_1$ eine der Gruppen $OR_2$ oder $SR_2$ repräsentiert; wobei $R_2$ für$C_1$–$C_8$-Alkyl, $C_3$-$C_8$-Alkenyl, $C_3$-$C_8$-Alkinyl oder für einen durch Hydroxy, Halogen, Cyano, Methoxycarbonyl, Ethoxycarbonyl, Methoxy, Ethoxy, Trimethoxysilyl, Triethoxysilyl, $N(CH_3)_2$ oder $N(C_2H_5)_2$ einfach substituierten $C_1$–$C_4$-Alkylrest oder für einen unsubstituierten oder ein- bis dreifach durch Halogen, Cyano, Methyl, Ethyl, Methoxy, Ethoxy, $CF_3$,

$C_1$-$C_2$-Haloalkoxy oder $N(C_1$-$C_2$-Alkyl$)_2$ substituierten Rest aus der Reihe Phenyl, Phenethyl, Benzyl, $C_5$-$C_6$-Cycloalkyl und Furfuryl steht.

Gruppe Ia: Verbindungen der Formel I, worin A für Phenyl steht; R Halogen, Cyano, Methyl, Ethyl, Methoxy, Ethoxy, $CF_3$, $C_1$-$C_2$-Haloalkoxy, $N(CH_3)_2$ oder $N(C_2H_5)_2$ bedeutet; n für 1, 2 oder 3 steht; $R_1$ eine der Gruppen $OR_2$ oder $SR_2$ repräsentiert; wobei $R_2$ für $C_1$-$C_8$-Alkyl, $C_3$-$C_8$-Alkenyl, $C_3$-$C_8$-Alkinyl oder für einen unsubstituierten oder ein- bis dreifach durch Halogen, Cyano, Methyl, Ethyl, Methoxy, Ethoxy, $CF_3$, $C_1$-$C_2$-Haloalkoxy oder $N(C_1$-$C_2$-Alkyl$)_2$ substituierten Rest aus der Reihe Phenyl, Benzyl, $C_5$-$C_6$-Cycloalkyl und Furfuryl steht.

Gruppe Ib: Verbindungen der Formel I, worin A für Phenyl steht; R Fluor, Chlor, Brom, Methyl, Methoxy oder $CF_3$ bedeutet; n für 1 oder 2 steht; $R_1$ für $OR_2$ oder $SR_2$ steht; wobei $R_2$ $C_1$-$C_5$-Alkyl, $C_3$-$C_4$-Alkenyl, $C_3$-$C_4$-Alkinyl, Cyclopentyl, Cyclohexyl, Furfuryl oder einen unsubstituierten oder ein- bis dreifach durch Fluor, Chlor, Brom, Methyl, Methoxy oder $CF_3$ substituierten Rest aus der Reihe Phenyl und Benzyl bedeutet.

Gruppe Ic: Verbindungen innerhalb der Gruppe Ib, worin n für 2 steht, R für Fluor oder Chlor steht; die an den 2- und 4-Positionen des Phenylrings substituiert sind; $R_1$ für $OR_2$ oder $SR_2$ steht; wobei $R_2$ für $C_1$-$C_5$-Alkyl, Allyl, Propargyl, Cyclohexyl, Benzyl oder unsubstituiertes oder ein- bis dreifach durch Fluor, Chlor, Brom, Methyl, Methoxy oder $CF_3$ substituiertes Phenyl bedeutet. Aliphatische Reste sind für den Substituenten $R_2$ bevorzugt.

Gruppe I-D: Verbindungen der Formel I, worin A für Phenoxyphenyl steht; n für 0, 1, 2, 3, 4 oder 5 steht; und die übrigen Substituenten wie in der Gruppe I-A definiert sind.

Gruppe Id: Verbindungen der Formel I innerhalb der Gruppe ID, worin die übrigen Substituenten wie in der Gruppe Ia definiert sind.

Gruppe Ie: Verbindungen der Formel I innerhalb der Gruppe Id, worin A für (p-Phenoxy)phenyl steht.

Gruppe If: Verbindungen der Formel I innerhalb der Gruppe Ie, worin R, $R_1$ und $R_2$ wie in Gruppe Ib definiert sind und n für 0, 1, 2, 3 oder 4 steht.

Gruppe Ig: Verbindungen der Formel I innerhalb der Gruppe If, worin $R_1$ und $R_2$ wie in Gruppe Ic definiert sind, n für 0, 1, 2, oder 3 steht und R Fluor, Chlor, Methyl, Methoxy oder $CF_3$ bedeutet.

Gruppe I-H: Verbindungen der Formel I, worin A für Phenyl oder p-Phenoxyphenyl steht; n 0, 1, 2 oder 3 bedeutet, R Halogen, Cyano, Methyl, Ethyl, Methoxy, Ethoxy, Methoxycarbonyl, Ethoxycarbonyl, $CF_3$ oder $C_1$-$C_2$ Haloalkoxy darstellt, $R_1$ die Gruppe $N(R_3)(R_4)$ bedeutet und $R_3$ und $R_4$ unabhängig voneinander für Wasserstoff oder $C_1$-$C_4$-Alkyl stehen oder gemeinsam mit dem Aminstickstoff einen Pyrrolidin-, Piperidin-, Piperazin- oder Morpholin-Ring bilden.

Gruppe I-I: Verbindungen innerhalb der Gruppe I-H, worin R Fluor, Chlor oder Methyl darstellt.

Gruppe I-J: Verbindungen der Formel I, worin A für p-Biphenyl steht, n = 0 ist, $R_1$ entweder $OR_2$ oder $SR_2$ bedeutet und $R_2$ $C_1$-$C_4$-Alkyl, Allyl oder Propargyl ist.

Besonders bevorzugte Einzelsubstanzen sind beispielsweise:

5-[2,4-Dichlorbenzyl-C-(tert.butylthio)imino] pyrimidin (Nr. 1.1);
5-[4-(p-Chlorphenoxy)-2-methylbenzyl-C-(tert.butylthio)imino]pyrimidin (Nr. 2.15);
5-[4-(p-Chlorphenoxy)-2-methylbenzyl-C-(isopropoxy)imino]pyrimidin (Nr. 2.16);
5-[4-(p-Chlorphenoxy)-2-chlorbenzyl-C-(tert.butylthio)imino]pyrimidin (Nr. 2.12);
5-[4-(p-Chlorphenoxy)-2-chlorbenzyl-C-(isopropoxy)imino]pyrimidin (Nr. 2.11).
5-[2,4-Dichlorbenzyl-C-(isopropoxy)imino]pyrimidin (Nr. 1.12)
5-[2,4-Dichlorbenzyl-C-(tert.butoxy)imino]pyrimidin (Nr. 1.13)
5-[2,4-Dichlorbenzyl-C-(allyloxy)imino]pyrimidin (Nr. 1.88)
5-[2,4-Dichlorbenzyl-C-(n-propoxy)imino]pyrimidin (Nr. 1.99)
5-[4-Chlorbenzyl-C-(tert.butylthio)imino]pyrimidin (Nr. 1.17)
5-[2,4-Dichlorbenzyl-C-(trifluorethoxy)imino]pyrimidin (Nr. 1.14)
5-[2,4-Dichlorbenzyl-C-(diethylamino)imino]pyrimidin (Nr. 1.95)
5-[2,4-Dichlorbenzyl-C-(tert.butylamino)imino] pyrimidin (Nr. 1.110)

Die Verbindungen der Formel I werden erfindungsgemäss dadurch hergestellt, dass man eine Verbindung der Formel II

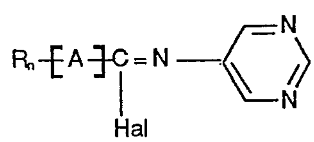

(II)

in Gegenwart einer Base mit einer Verbindung der Formel III

$$R_1\text{-}H \qquad \text{(III)}$$

umsetzt. Dabei haben $R_n$, A und $R_1$ in den Formeln II und III die unter Formel I angegebenen Bedeutungen, Hal steht für Halogen, vorzugsweise für Chlor oder Brom.

Die Umsetzung von (II) mit (III) zu (I) erfolgt unter Halogenwasserstoffabspaltung. Günstige Reaktionstemperaturen liegen zwischen 0° und +180°C, vorzugsweise +20° und +150°C bzw. am Siedepunkt des Lösungsmittels bzw. Lösungsmittelgemisches. Im allgemeinen ist die Verwendung von säurebindenden Mitteln bzw. Kondensationsmitteln (Basen) vorteilhaft. Als solche kommen organische und anorganische Basen in Betracht, z. B. tertiäre Amine wie Trialkylamine (Trimethylamin, Triethylamin, Tripropylamin usw.), Pyridin und Pyridinbasen (4-Dimethylaminopyridin, 4-Pyrrolidylaminopyridin usw.), Alkoholate wie z. B. Kaliumtert.-Butylat, Oxide und Hydroxide, Carbonate und Hydrogencarbonate

von Alkali- und Erdalkalimetallen sowie die Salze der Essigsäure wie z. B. die Alkaliacetate.

Bei der Herstellung aller hierin genannten Ausgangs-, Zwischen- und Endprodukte können grundsätzlich, sofern nicht ausdrücklich im einzelnen spezifiziert, ein oder mehrere reaktionsinerte Lösungs- oder Verdünnungsmittel anwesend sein. In Frage kommen beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether, und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; Dimethylsulfoxid; Ketone wie Aceton, Diethylketon, Methylethylketon und Gemische solcher Lösungsmittel untereinander.

Die Verbindungen der Formel III sind allgemein bekannt oder lassen sich nach an sich bekannten Methoden herstellen.

Die Verbindungen der Formel II sind neu. Aufgrund ihrer Struktur sind sie geradezu prädestiniert für die Herstellung der mikrobizid, aktiven Endprodukte der Formel I oder anderer Pestizide. Sie sind somit wertvolle Zwischenprodukte und bilden einen wichtigen Bestandteil der vorliegenden Erfindung.

Die Herstellung von Verbindungen der Formel II kann z. B. folgendermassen erfolgen:
5-Aminopyrimidin der Formel IV

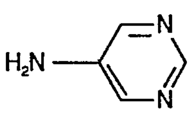

(IV)

wird vorzugsweise in Gegenwart einer Base mit einem Säurehalogenid, vorzugsweise dem Chlorid oder Bromid der Formel V

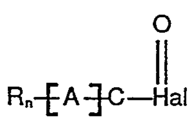

(V)

zu einer Verbindung der Formel VI

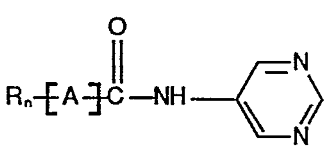

(VI)

N-acyliert und letztere, auf an sich bekannte Weise (z. B. mit $SOCl_2$, $PBr_5$, $PCl_5$, N-Bromsuccinimid usw.) zu einer Verbindung der Formel II halogeniert. Dabei haben die Substituenten $R_n$ und A in den Formeln V und VI die unter Formel I angegebenen Bedeutungen und Hal steht für Halogen, vorzugsweise Chlor oder Brom. Die Umsetzung von (IV) mit (V) zu (VI) erfolgt unter den für die Reaktion von (II) mit (III) zu (I) genannten Bedingungen.

Bei der Herstellung der Verbindungen der Formel I kann man nach einer zweiten Herstellungsvariante auch die Verbindung der Formel VI vorlegen, diese durch Halogenierung (z. B. mit $SOCl_2$) in das Halogenid der Formel (II) (Chlorid) überführen und (II) in situ, erfindungsgemäss zu (I) umsetzen. Dieses Eintopfverfahren ist ebenfalls Bestandteil dieser Erfindung, da das Zwischenprodukt II hierbei intermediär auftritt und, falls gewünscht, aus dem Reaktionsmedium isoliert werden kann. Die Verbindungen der Formel VI sind somit unmittelbare Vorprodukte bei der Herstellung der Verbindungen der Formel I. Sie sind neu und überdies selbst mikrobizid wirksam. Sie zeigen u. a. sehr gute pflanzenfungizide Wirkung und sind z. B. auf dem gleichen Indikationsgebiet wie die Endprodukte der Formel I einsetzbar. Die Verbindungen der Formel VI sind ein Bestandteil vorliegender Erfindung.

5-Aminopyrimidin ist aus der Literatur bekannt (vgl. Whittaker, J. Chem. Soc. 1951, p. 1568). Desgleichen sind die Säurehalogenide der Formel V bekannt oder lassen sich nach an sich bekannten Methoden bzw. analog zu den bekannten Vertretern herstellen.

Typische Vertreter der Formel II sind im Rahmen der vorliegenden Erfindung z. B. folgende Verbindungen:

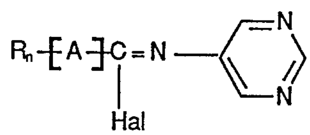

(II)

wobei diese Aufzählung keinen limitierenden Charakter hat:
(Temperaturen sind in Celsiusgraden angegeben)

| Verb. Nr. | $R_n$-[A]- | Hal | |
|---|---|---|---|
| 1 | $C_6H_3Cl_2(2,4)$ | Cl | Hydrochlorid, Smp. 132–136° (Zers.) |
| 2 | $C_6H_3Cl_2(2,4)$ | Br | |
| 3 | $C_6H_4Cl(4)$ | Cl | Hydrochlorid, Smp. 136–138° (Zers.) |
| 4 | $C_6H_4Cl(4)$ | Br | |
| 5 | $C_6H_4F(4)$ | Cl | |
| 6 | $C_6H_4F(4)$ | Br | |
| 7 | $C_6H_4F(3)$ | Cl | Smp. 81–82° |
| 8 | $C_6H_4F(3)$ | Br | |
| 9 | $C_6H_4F(2)$ | Cl | |
| 10 | $C_6H_4F(2)$ | Br | |
| 11 | $C_6H_4Cl(2)$ | Cl | |
| 12 | $C_6H_4Cl(2)$ | Br | |
| 13 | $C_6H_4Br(4)$ | Cl | |
| 14 | $C_6H_4Br(4)$ | Br | |
| 15 | $C_6H_3Br_2(2,5)$ | Cl | |
| 16 | $C_6H_3Br_2(2,5)$ | Br | |
| 17 | $C_6H_3Cl_2(2,6)$ | Cl | |
| 18 | $C_6H_3Cl_2(2,6)$ | Br | |
| 19 | $C_6H_3F_2(2,6)$ | Cl | |
| 20 | $C_6H_3F_2(2,6)$ | Br | |
| 21 | $C_6H_4J(2)$ | Cl | |

| Verb. Nr. | $R_n$-[A]- | Hal | |
|---|---|---|---|
| 22 | $C_6H_4J(2)$ | Br | |
| 23 | $C_6H_4J(3)$ | Cl | |
| 24 | $C_6H_3J_2(2,3)$ | Cl | |
| 25 | $C_6H_3J_2(3,4)$ | Cl | |
| 26 | $C_6H_4CH_3(4)$ | Cl | |
| 27 | $C_6H_4CH_3(4)$ | Br | |
| 28 | $C_6H_3(CH_3)_2(2,4)$ | Cl | |
| 29 | $C_6H_3(CH_3)_2(3,4)$ | Cl | |
| 30 | $C_6H_4CF_3(4)$ | Cl | |
| 31 | $C_6H_4CH_3(3)$ | Cl | |
| 32 | $C_6H_3CH_3(2)NO_2(4)$ | Cl | |
| 33 | $C_6H_2CH_3(3)(NO_2)_2(3,5)$ | Cl | |
| 34 | $C_6H_3CH_3(5)NO_2(2)$ | Cl | |
| 35 | $C_6H_3(CH_3)_2(2,3)$ | Cl | |
| 36 | $C_6H_2J_3(2,3,5)$ | Cl | |
| 37 | $C_6H_4[C(CH_3)_3](4)$ | Cl | |
| 38 | $C_6H_4[N(CH_3)_2](4)$ | Cl | |
| 39 | $C_6H_4[N(CH_3)_2](3)$ | Cl | |
| 40 | $C_6H_4OCH_3(3)$ | Cl | |
| 41 | $C_6H_4OCH_3(2)$ | Cl | |
| 42 | $C_6H_3(OCH_3)_2(3,5)$ | Cl | |
| 43 | $C_6H_3(OCH_3)_2(3,4)$ | Cl | |
| 44 | $C_6H_3(OCH_3)(2,4)$ | Cl | |
| 45 | $C_6H_4C_6H_5(4)$ | Cl | |
| 46 | $C_6H_4C_6H_5(4)$ | Br | |
| 47 | $C_6H_4(CH_2C_6H_5)(4)$ | Cl | |
| 48 | $C_6H_4(CH_2C_6H_5)(4)$ | Br | |
| 49 | α-Naphthyl | Cl | |
| 50 | α-Naphthyl | Br | |
| 51 | β-Naphthyl | Cl | |
| 51a | $C_6H_4Cl(2)$, Br(4) | Cl | Öl |
| 51b | $C_6H_4F(2)$, Cl(4) | Cl | Öl |

sowie Verbindungen der Formel II, worin $R_n$-[A]- für

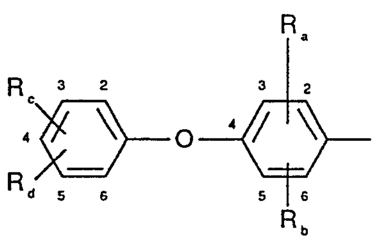

steht:

| Verb. Nr. | $R_a$ | $R_b$ | $R_c$ | $R_d$ | Hal | |
|---|---|---|---|---|---|---|
| 52 | H | H | H | H | Cl | Öl |
| 53 | H | H | H | H | Br | |
| 54 | 2-Cl | H | H | H | Cl | |
| 55 | 2-Cl | H | H | H | Br | |
| 56 | 2-$CH_3$ | H | H | H | Cl | |
| 57 | 2-$CH_3$ | H | H | H | Br | |
| 58 | H | H | 4-Cl | H | Cl | Öl |
| 59 | H | H | 4-Cl | H | Br | |
| 60 | H | H | 4-$CH_3$ | H | Cl | |
| 61 | H | H | 4-$CH_3$ | H | Br | |
| 62 | 2-Cl | H | 4-Cl | H | Cl | |
| 63 | 2-Cl | H | 4-Cl | H | Br | |
| 64 | 2-$CH_3$ | H | 4-$CH_3$ | H | Cl | |
| 65 | 2-$CH_3$ | H | 4-$CH_3$ | H | Br | |
| 66 | 2-Cl | H | 4-$CH_3$ | 4-$CH_3$ | Cl | |
| 67 | 2-Cl | H | 4-$CH_3$ | H | Br | |
| 68 | 2-$CH_3$ | H | 4-Cl | H | Cl | |
| 69 | 2-$CH_3$ | H | 4-Cl | H | Br | |
| 70 | 2-Cl | 3-Cl | 4-Cl | 2-Cl | Cl | |
| 71 | 2-Cl | 3-Cl | 4-$CH_3$ | H | Cl | |
| 72 | 2-$CH_3$ | H | 4-Cl | 2-Cl | Cl | |
| 73 | H | H | 4-$CF_3$ | H | Cl | |
| 74 | H | H | 4-$OCH_3$ | H | Cl | |

Typische Vertreter von Verbindungen der Formel VI

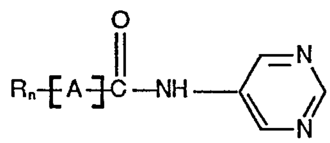

(VI),

sind im Rahmen der vorliegenden Erfindung z. B.

| Verb. Nr. | $R_n$-[A]- | Phys. Konst. |
|---|---|---|
| 75 | $C_6H_4NO_2(4)$ | Smp. 193–194° |
| 76 | $C_6H_3Cl(2,4)$ | Smp. 139–140° |
| 77 | $C_6H_4Cl(4)$ | Smp. 177–179° |
| 78 | $C_6H_4F(4)$ | Smp. 148–150° |
| 79 | $C_6H_4F(3)$ | Smp. 162–163° |
| 80 | $C_6H_4F(2)$ | Smp. 125–126° |
| 81 | $C_6H_4Cl(2)$ | |
| 82 | $C_6H_4Br(4)$ | Smp. 194–196° |
| 83 | $C_6H_3Br_2(2,5)$ | |
| 84 | $C_6H_3Cl_2(2,6)$ | |
| 85 | $C_6H_3F_2(2,6)$ | |
| 86 | $C_6H_4J(2)$ | Harz |
| 87 | $C_6H_4J(3)$ | |
| 88 | $C_6H_3J_2(2,3)$ | |
| 89 | $C_6H_4CH_3(4)$ | Smp. 150–153° |
| 90 | $C_6H_3(CH_3)_2(2,4)$ | |
| 91 | $C_6H_3(CH_3)_2(3,4)$ | |
| 92 | $C_6H_4CF_3(4)$ | |
| 93 | $C_6H_4CH_3(3)$ | |
| 94 | $C_6H_3CH_3(2)NO_2(4)$ | |
| 95 | $C_6H_2CH_3(3)(NO_2)_2(3,5)$ | |
| 96 | $C_6H_3CH_3(5)NO_2(2)$ | |
| 97 | $C_6H_3(CH_3)_2(2,3)$ | |
| 98 | $C_6H_2J_3(2,3,5)$ | |
| 99 | $C_6H_4[C(CH_3)_3](4)$ | Smp. 214–216° |
| 100 | $C_6H_4[N(CH_3)_2](3)$ | |
| 101 | $C_6H_4OCH_3(3)$ | |
| 102 | $C_6H_3(OCH_3)_2(3,5)$ | |
| 103 | $C_6H_3(OCH_3)_2(3,4)$ | |
| 104 | $C_6H_3(OCH_3)_2(2,4)$ | |
| 105 | $C_6H_4C_6H_5(4)$ | |
| 106 | $C_6H_4(CH_2C_6H_5)(4)$ | |
| 107 | α-Naphthyl | |
| 108 | β-Naphthyl | |
| 108a | $C_6H_2(OCH_3)_3(3,4,5)$ | Smp. 167–169° |
| 108b | $C_6H_5$ | Smp. 140–143° |
| 108c | $C_6H_4NH_2(4)$ | Smp. 247–248° |
| 108d | $C_6H_4OCH_3(4)$ | Smp. 217–218° |

| Verb. Nr. | $R_n$-[A]- | Phys. Konst. |
|---|---|---|
| 108e | $C_6H_4CF_3(2)$ | Smp. 154-156° |
| 108f | $C_6H_4F(2)$, Cl(4) | Smp. 138-141° |
| 108g | $C_6H_4Cl(2)$, Br(4) | Smp. 145-150° |

sowie Verbindungen der Formel VI, worin $R_n$-[A]-für

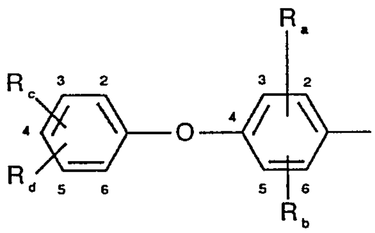

steht:

| Verb. Nr. | $R_a$ | $R_b$ | $R_c$ | $R_d$ | Phys. Konstante |
|---|---|---|---|---|---|
| 109 | H | H | H | H | Smp. 155–158° |
| 110 | 2-Cl | H | H | H | |
| 111 | 2-$CH_3$ | H | H | H | |
| 112 | H | H | 4-Cl | H | Smp. 196–199° |
| 113 | H | H | 4-$CH_3$ | H | |
| 114 | 2-Cl | H | 4-Cl | H | Smp. 211–212° |
| 115 | 2-$CH_3$ | H | 4-$CH_3$ | H | |
| 116 | 2-Cl | H | 4-$CH_3$ | H | |
| 117 | 2-$CH_3$ | H | 4-Cl | H | Smp. 157–159° |
| 118 | 2-Cl | 3-Cl | 4-Cl | 2-Cl | |
| 119 | 2-Cl | 3-Cl | 4-$CH_3$ | H | |
| 120 | 2-$CH_3$ | H | 4-Cl | 2-Cl | |
| 121 | H | H | 4-$CH_3$ | 2-Cl | |
| 122 | H | H | 4-$CF_3$ | H | |
| 123 | H | H | 4-$OCH_3$ | H | |
| 124 | 2-$OCH_3$ | H | H | H | |
| 125 | 2-$OCH_3$ | H | 4-$OCH_3$ | H | |
| 126 | H | H | 4-$OCH_3$ | 2-$OCH_3$ | |

Analog zur C=C-Doppelbindung führt die C=N-Doppelbindung in den Verbindungen der Formel I, aber auch in den Zwischenprodukten der Formel II, zu unterschiedlichen geometrischen Isomeren:

Verbindungen der Formel I

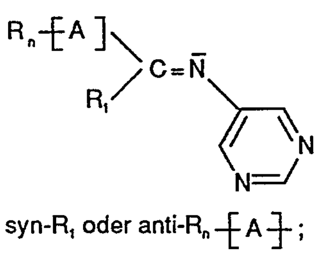

(I)

syn-$R_1$ oder anti-$R_n$-[A]-;

(trans-Form)

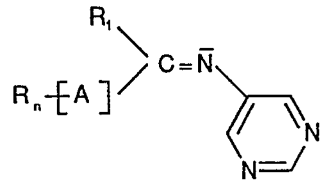

syn-$R_n$ -[A]- oder anti-$R_1$

(cis-Form)

Verbindungen der Formel II

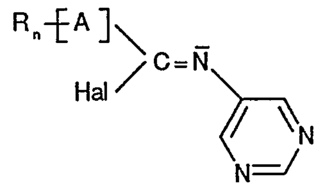

syn-Hal oder anti-$R_n$ -[A]-;

(trans-Form)

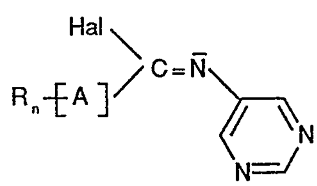

syn-$R_n$ -[A]- oder anti-Hal

(cis-Form)

Im allgemeinen entsteht bei der Herstellung von Verbindungen der Formeln I und II ein Gemisch der cis- und trans-Form, wobei die thermodynamisch günstigere Form bevorzugt gebildet wird. Die Substanzen können ohne Auftrennung der Isomeren im Pflanzenschutz eingesetzt werden. Allerdings kann der Einsatz der reinen Isomeren zu einer Wirkungserhöhung führen.

Die Erfindung betrifft somit sämtliche isomeren Verbindungen der Formel I in reiner Form oder in beliebigem Zahlenverhältnis zueinander.

Das Herstellungsverfahren von Verbindungen der Formel I ist in seinen beschriebenen Varianten ein Bestandteil dieser Erfindung.

Es wurde überraschend festgestellt, dass Verbindungen der Formel I ein für praktische Bedürfnisse sehr günstiges Mikrobizid-Spektrum gegen phytopathogene Pilze und Bakterien aufweisen. Sie besitzen sehr vorteilhafte kurative, präventive und systemische Eigenschaften und lassen sich zum Schutz von Kulturpflanzen einsetzen. Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben.

Die Wirkstoffe sind gegen die den folgenden Klassen angehörenden phytopathogenen Pilze wirksam: Ascomyceten (z.B. Venturia, Podosphaera, Erysiphe, Monilinia, Uncinula); Basidiomyceten (z.B. die Gattungen Hemileia, Rhizoctonia, Puccinia); Fungi imperfecti (z.B. Botrytis, Helmintosporium, Fusarium, Septoria, Cercospora und Alternaria). Überdies wirken die Verbindungen der Formel I systemisch. Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden. Die erfindungsgemässen Wirkstoffe zeichnen sich durch besonders gute Pflanzenverträglichkeit aus.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen.

Darüberhinaus schliesst die vorliegende Erfindung auch die Herstellung agrochemischer Mittel ein, die gekennzeichnet ist durch das innige Vermischen der Aktivsubstanz mit einem oder mehreren hierin beschriebenen Trägermaterialien. Eingeschlossen ist auch ein Verfahren zur Behandlung von Pflanzen, das sich durch Applikation der Verbindungen der Formel I bzw. der neuen Mittel auszeichnet.

Als Zielkulturen für die hierin offenbarten Indikationsgebiete gelten im Rahmen dieser Erfindung z.B. folgende Pflanzenarten: Getreide: (Weizen, Gerste, Roggen, Hafer, Reis, Sorghum und Verwandte); Rüben: (Zucker- und Futterrüben); Kern-, Stein- und Beerenobst: (Äpfel, Birnen, Pflaumen, Pfirsiche, Mandeln, Kirschen, Erd-, Him- und Brombeeren); Hülsenfrüchte: (Bohnen, Linsen, Erbsen, Soja); Ölkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Blumen, Sträucher, Laubbäume und Nadelbäume wie Koniferen). Diese Aufzählung stellt keine Limitierung dar.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I ist das Aufbringen auf das Blattwerk (Blattapplikation) oder in den Boden (Bodenapplikation). Die Verbindungen der Formel I können aber auch auf Samenkörner aufgebracht werden (Coating), indem man die Körner entweder in einer flüssigen Zubereitung des Wirkstoffs tränkt oder sie mit einer festen Zubereitung beschichtet.

Die Verbindungen der Formel I werden dabei in unveränderter Form oder vorzugsweise zusammen mit den in der Formulierungstechnik üblichen Hilfsmittel eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt 100 g bis 2 kg AS/ha, insbesondere bei 100 g bis 600 g AS/ha.

Als Lösungsmittel können in Frage kommen: Kohlenwasserstoffe, z.B. Xylolgemische, Hexan oder Cyclohexan, Alkohole und Glykole sowie deren Ether und Ester, wie Ethanol, Ethylenglykol, Ethylenglykolmonomethyl- oder Ethylether, Ketone wie Cyclohexanon, polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Besonders vorteilhafte, applikationsfördernde Zuschlagstoffe sind ferner natürliche oder synthetische Phospholipide aus der Reihe der Kephaline und Lecithine, wie z.B. Phosphatidylethanolamin, Phosphatidylserin, Phosphatidylglycerin, Lysolecithin.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können z.B. wasserlösliche Seifen (Fettsäure-Salze) sein.

Häufiger werden sogenannte synthetische Tenside verwendet, insbesondere Fettsulfonate, Fettsulfate, sulfonierte Benzimidazolderivate oder Alkylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen.

Die in der Formulierungstechnik gebräuchlichen Tenside sind u.a. in folgenden Publikationen beschrieben:

«Mc Cutcheon's Detergents and Emulsifiers Annual» BC Publishing Corp., Ridgewood New Jersey, 1981;

Helmut Stache «Tensid-Taschenbuch» Carl Hanser-Verlag München/Wien 1981.

Die agrochemischen Zubereitungen enthalten in der Regel 0,1 bis 99%, insbesondere 0,1 bis 95% Wirkstoff der Formel I, 99,9 bis 1%, insbesondere 99,8 bis 5% eines festen oder flüssigen Zusatzstoffes und 0 bis 25%, insbesondere 0,1 bis 25% eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe einzuschränken. Temperaturen sind in Celsiusgraden angegeben. Prozente und Teile beziehen sich auf das Gewicht. Darüberhinaus werden folgende Symbole verwendet: h = Stunde; d = Tag; min = Minute; RT = Raumtemperatur; N = Normalität; abs = absolut, wasserfrei, DMSO = Dimethylsulfoxid; DMF = Dimethylformamid. Druckangaben erfolgen in Millibar mb, oder Bar b. $C_6H_5$ charakterisiert stets eine Phenylgruppe. Eine substituierte Phenylgruppe z.B. ortho-, para-Dichlorphenyl wird dann dementsprechend als $C_6H_3Cl_2$ (2,4) abgekürzt.

Herstellungsbeispiele

Beispiel H1: Herstellung von

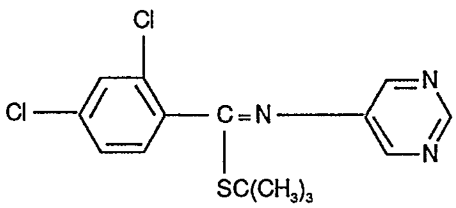

5-[2,4-Dichlorbenzyl-C-(tert.butylthio)imino]pyrimidin

a) Herstellung der Zwischenstufe

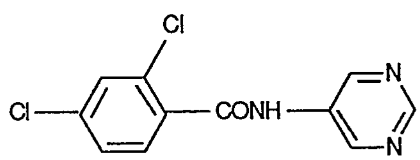

N-(2,4-Dichlorbenzoyl)-5-aminopyrimidin

Zu einer Lösung von 3,26 g (0,0343 Mol) 5-Aminopyrimidin in 20 ml Tetrahydrofuran und 20 ml Pyridin lässt man bei 50°C eine Lösung von 8,62 g (0,041 Mol) 2,4-Dichlorbenzylchlorid in 10 ml Tetrahydrofuran zutropfen, wobei sich Pyridinhydrochlorid abscheidet. Man gibt Wasser zu und extrahiert unter Zusatz von 20 ml konz. HCl bei pH 1 mit Chloroform. Nach dem Trocknen des Extrakts mit $Na_2SO_4$ erhält man beim Einengen 10,9 g Rohprodukt, das aus 50 ml Toluol und 10 ml Essigester umkristallisiert wird. Ausbeute 2,58 (28,1% d.Th.); Smp. 139-141°C. Beim Einengen des Filtrats kristallisieren weitere 2,49 g (27,1% d.Th.) Produkt mit Smp. 137-139°C aus. Das Filtrat wird eingeengt und der Rückstand über eine Kieselgelsäule (Laufmittel 2 Teile Essigester/1 Teil Hexan) chromatographiert; man erhält weitere 2,05 g (22,3% d.Th.) Substanz.

b) Herstellung von N-(5-Pyrimidyl)-2,4-dichlorbenzimidchlorid

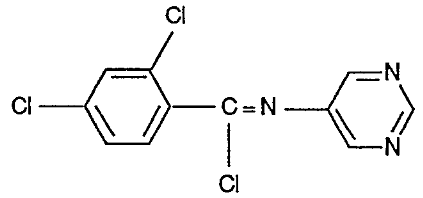

10,7 g (0,04 Mol) N-(2,4-Dichlorbenzoyl)-5-aminopyrimidin werden mit 10 g Phosphorpentachlorid allmählich auf 120°C erhitzt, wobei unter HCl-Entwicklung eine Lösung entsteht. Nach einer Stunde verdünnt man mit 1 ml Toluol und 15 ml Cyclohexan und lässt unter Rühren erkalten. Dabei kristallisiert N-(5-Pyrimidyl)-2,4-dichlorbenzimidchlorid als Hydrochlorid aus, das abgesaugt, mit Cyclohexan gewaschen und im Exsikkator über $P_2O_5$ getrocknet wird. Die Ausbeute beträgt 5,5 g; Smp. 132–136°C (Zers.).

c) Herstellung des Endproduktes

10,6 g (0,0395 Mol) n-(2,4-Dichlorbenzoyl)-5-aminopyrimidin werden mit 30 ml Thionylchlorid 2 Stunden am Rückfluss gekocht. Einengen der Lösung im Vakuum am Rotationsverdampfer ergibt als Zwischenprodukt das N-(5-Pyrimidyl)-2,4-dichlorbenzimidchlorid, welches zur weiteren Umsetzung sofort in 100 ml Pyridin gelöst, und mit 8,8 ml tert. Butylmercaptan 3 Stunden auf 100°C gehalten wird. Danach giesst man die Lösung auf 700 ml Eiswasser und extrahiert mit Essigester. Nach dem Waschen des Extrakts mit Wasser, dem Trocknen mit $Na_2SO_4$ und dem Einengen erhält man 10,8 g Rohprodukt, das mittels einer Kieselgelsäule (Laufmittel 2 Teile Petroläther/1 Teil Essigester) gereinigt wird. Man erhält 8,4 g (62,5% d.Th.) Substanz, die nach der Umkristallisation aus einem Gemisch von Toluol und Cyclohexan einen Smp. von 108–109°C aufweist.

Beispiel H2: Herstellung von

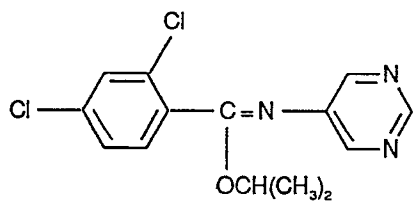

5-[2,4-Dichlorbenzyl-C-(isopropoxy)imino]pyrimidin (Verb. 1.12)

Einer Lösung von 2,9 ml 2-Propanol (0,037 Mol) in 30 ml Tetrahydrofuran gibt man in Portionen 2,1 g 55%iges Natriumhydrid in Öl zu, wobei sich Na-Isopropylat abscheidet. Sodann lässt man eine Lösung von 0,025 Mol rohem N-(5-Pyrimidyl)-2,4-dichlorbenzimidchlorid in 30 ml Tetrahydrofuran (Herstellung siehe Beispiel H1c) unter Kühlung bei −10°C zutropfen. Das Reaktionsgemisch wird dann bei Raumtemperatur auf Eiswasser gegossen und mit Ethylacetat extrahiert. Nach dem Trocknen der organischen Phase mit $Na_2SO_4$ und dem Einengen erhält man ein Rohprodukt, das mittels einer Chromatographiesäule über Kieselgel mit einem Gemisch aus 3 Teilen Hexan und 1 Teil Ethylacetat gereinigt wird. Man erhält so 4,8 g Substanz, die nach der Umkristallisation 3,9 g reines Produkt vom Smp. 85–86°C ergibt.

Beispiel H3: Herstellung von

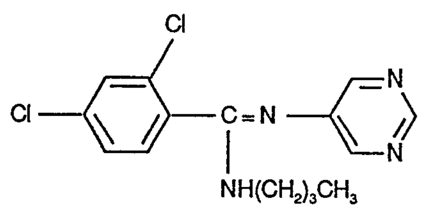

5-[2,4-Dichlorbenzyl-C-(n-butylamino)imino]pyrimidin (Verb. 1.108)

0,02 Mol rohes N-(5-Pyrimidyl)-2,4-dichlorbenzimidchlorid (Herstellung siehe Beispiel H1c) werden in 30 ml Dioxan gelöst und mit 5,9 ml n-Butylamin 1 Stunde auf 80°C erhitzt. Nach dem Erkalten wird mit Methylenchlorid und Wasser extrahiert, die organische Phase mit $Na_2SO_4$ getrocknet und sodann eingeengt. Die Reinigung des Rohprodukts (7,4 g) mittels einer Chromatographiesäule über Kieselgel unter Verwendung von Ethylacetat als Laufmittel ergibt 5,7 g Reinsubstanz als Öl $n_D^{40}$ 1,5810.

Analog zu den beschriebenen Arbeitsweisen erhält man auch die nachfolgend genannten Verbindungen der Formel I:

Tabelle 1: Verbindungen der Formel

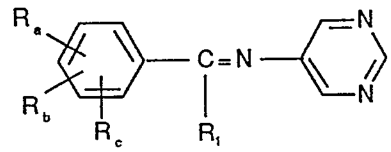

| Verb. Nr. | $R_a$ | $R_b$ | $R_c$ | $R_1$ | Physik. Konst. [°C] |
|---|---|---|---|---|---|
| 1.1 | 4-Cl | 2-Cl | H | $SC(CH_3)_3$ | Smp. 108-109° |
| 1.2 | 4-Cl | 2-Cl | H | $SCH_3$ | Smp. 85-87° |
| 1.3 | 4-Cl | 2-Cl | H | $SCH(CH_3)_2$ | Smp. 76-78° |
| 1.4 | 4-Cl | 2-Cl | H | $SCH_2CH_2CH_3$ | $n_D^{40}$ 1.6123 |
| 1.5 | 4-Cl | 2-Cl | H | $S(CH_2)_9CH_3$ | |
| 1.6 | 4-Cl | 2-Cl | H | $SCH_2CH_2N(C_2H_5)_2$ | |
| 1.7 | 4-Cl | 2-Cl | H | 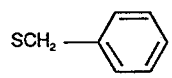 | $n_D^{48}$ 1.6465 |
| 1.8 | 4-Cl | 2-Cl | H | $SCH_2CH{=}CH_2$ | |
| 1.9 | 4-Cl | 2-Cl | H | 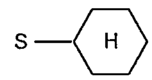 | Smp. 86-88° |
| 1.10 | 4-Cl | 2-Cl | H | 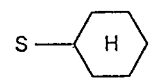 | |
| 1.11 | 4-Cl | 2-Cl | H | $OCH_3$ | $n_D^{24}$ 1.5965 |
| 1.12 | 4-Cl | 2-Cl | H | $OCH(CH_3)_2$ | Smp. 85-86° |
| 1.13 | 4-Cl | 2-Cl | H | $OC(CH_3)_3$ | Smp. 103-104° |
| 1.14 | 4-Cl | 2-Cl | H | $OCH_2CF_3$ | Smp. 96-98° |
| 1.15 | 4-Cl | 2-Cl | H | $OCH_2CH_2CN$ | |
| 1.16 | 4-Cl | 2-Cl | H | $OCH_2CH_2NO_2$ | |
| 1.17 | 4-Cl | H | H | $SC(CH_3)_3$ | Smp. 129-130° |
| 1.18 | 4-Cl | H | H | $SCH(CH_3)_2$ | |
| 1.19 | 4-Cl | H | H | $SCH_3$ | |
| 1.20 | 4-Cl | H | H | $SCH_2CH(CH_3)_2$ | |
| 1.21 | 4-Cl | H | H | $SCH_2CH_2OH$ | |
| 1.22 | 4-F | H | H | $SCH_3$ | |
| 1.23 | 4-F | H | H | $SC(CH_3)_3$ | Smp. 102-103° |
| 1.24 | 4-F | H | H | $SCH(CH_3)_2$ | $n_D^{28}$ 1.5910 |
| 1.25 | 3-F | H | H | $SC(CH_3)_3$ | Smp. 76° |
| 1.26 | 3-F | H | H | $SC_2H_5$ | $n_D^{25}$ 1.6075 |
| 1.27 | 2-F | H | H | $SCH(CH_3)_2$ | |
| 1.28 | 2-Cl | H | H | $SCH(CH_3)_2$ | |
| 1.29 | 4-Br | H | H | $SC(CH_3)_3$ | Smp. 120-121° |
| 1.30 | 4-Br | H | H | 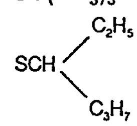 | $n_D^{26}$ 1.6065 |
| 1.31 | 2-Br | 5-Br | H | $SC(CH_3)_3$ | |
| 1.32 | 2-Cl | 5-Cl | H | $SC(CH_3)_3$ | |
| 1.33 | 2-Cl | 6-Cl | H | $SCH_3$ | |
| 1.34 | 2-F | 6-F | H | $SC_2H_5$ | |
| 1.35 | 2-Cl | 6-F | H | $SC(CH_3)_3$ | |
| 1.36 | 2-J | H | H | $SC_3H_7$ | |
| 1.37 | 3-J | H | H | $SC_2H_5$ | |
| 1.38 | 2-J | 3-J | 5-J | $SC_3H_7(n)$ | |
| 1.39 | 3-J | 4-J | 5-J | $SC_3H_7(n)$ | |
| 1.40 | 3-J | H | H | $SC_4H_9(n)$ | |
| 1.41 | 2-Cl | 4-Cl | H | $OCH_2{\equiv}CH$ | Öl |
| 1.42 | $2\text{-}CH_3$ | 4-Cl | H | 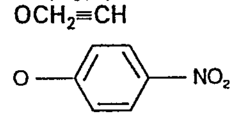 | |
| 1.43 | $4\text{-}CH_3$ | H | H | $OCH(CH_3)_2$ | $n_D^{24}$ 1.5618 |

| Verb. Nr. | $R_a$ | $R_b$ | $R_c$ | $R_1$ | Physik Konst. [°C] |
|---|---|---|---|---|---|
| 1.44 | 4–$CH_3$ | H | H | $SC_2H_5$ | $n_D^{24}$ 1.6183 |
| 1.45 | 4–$CH_3$ | H | H | 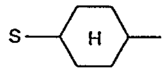 | $n_D^{23}$ 1.6080 |
| 1.46 | 4–$CH_3$ | 2–$CH_3$ | H | $N(CH_3)_2$ | |
| 1.47 | 4–$CH_3$ | 3–$CH_3$ | H | $OCH_2CH_2OCH_3$ | |
| 1.48 | 4–$CF_3$ | H | H | $SC_3H_7(n)$ | |
| 1.49 | 3–$CF_3$ | H | H | 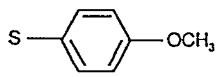 | |
| 1.50 | 2–$CF_3$ | H | H | $OCH_3$ | |
| 1.51 | 2–$CH_3$ | 4–$CH_3$ | 6–$CH_3$ | $SC_3H_7(n)$ | |
| 1.52 | 3–$CH_3$ | H | H | $OC_2H_5$ | |
| 1.53 | 2–$CH_3$ | 3–$NO_2$ | 5–$NO_2$ | $OCH_3$ | |
| 1.54 | 3–$CH_3$ | 4–$NO_2$ | H | $SCH(CH_3)_2$ | |
| 1.55 | 5–$CH_3$ | 2–$NO_2$ | H | $SCH_3$ | |
| 1.56 | 2–$CH_3$ | H | H | $OCH(CH_3)_2$ | |
| 1.57 | 2–$CH_3$ | 3–$CH_3$ | H | 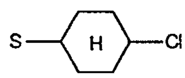 | |
| 1.58 | 2–$CH_3$ | 5–$CH_3$ | H | $SCH_2CH_2N(CH_3)_2$ | |
| 1.59 | 3–$CH_3$ | 5–$CH_3$ | H | 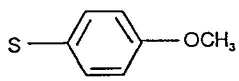 | |
| 1.60 | 4–$C(CH_3)_3$ | H | H | $SCH(CH_3)_2$ | Smp. 93–94° |
| 1.61 | 4–$C(CH_3)_3$ | H | H | $OCH_2CH(CH_3)_2$ | |
| 1.62 | 4–Cl | 3–$NO_2$ | 5–$NO_2$ | $OCH_3$ | |
| 1.63 | 4–CN | H | H | 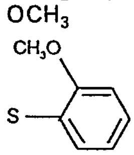 | |
| 1.64 | 2–$NO_2$ | 4–$NO_2$ | H | $N(C_3H_7)_2$ | |
| 1.65 | 2–$NO_2$ | 4–$NO_2$ | H | $OC_3H_7(n)$ | |
| 1.66 | 2–$NO_2$ | 4–$NO_2$ | H | $SCH_2CH_2OH$ | |
| 1.67 | 3–$NO_2$ | 4–$NO_2$ | H | 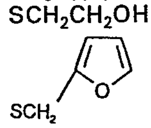 | |
| 1.68 | 3–$NO_2$ | 5–$NO_2$ | H | $O(CH_2)_9CH_3$ | |
| 1.69 | 4–$NO_2$ | H | H | $SC(CH_3)_3$ | |
| 1.70 | 4–$NO_2$ | H | H | $SC_4H_9(n)$ | $n_D^{23}$ 1.6138 |
| 1.71 | 2–$NO_2$ | H | H | 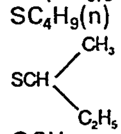 | |
| 1.72 | 2–$NO_2$ | H | H | $OCH_3$ | |
| 1.73 | 3–$NO_2$ | H | H | $SCH(CH_3)_2$ | |
| 1.74 | 3–$NO_2$ | H | H | $N(CH_3)_2$ | |
| 1.75 | 4–$N(CH_3)_2$ | H | H | 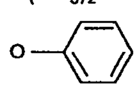 | |
| 1.76 | 4–$N(CH_3)_2$ | H | H | 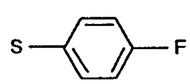 | |
| 1.77 | 3–$N(CH_3)_2$ | H | H | $SCH_3$ | |
| 1.78 | 3–$OCH_3$ | 5–$OCH_3$ | H | $SCH(CH_3)_2$ | |
| 1.79 | 3–$OCH_3$ | 4–$OCH_3$ | H | 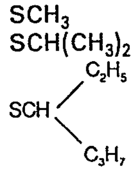 | |

| Verb. Nr. | $R_a$ | $R_b$ | $R_c$ | $R_1$ | Physik Konst. [°C] |
|---|---|---|---|---|---|
| 1.80 | 2-$OCH_3$ | 4-$OCH_3$ | H | $OC_5H_{11}$(n) | |
| 1.81 | 2-$OCH_3$ | 6-$OCH_3$ | H | 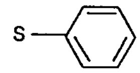 | |
| 1.82 | 2-$OCH_3$ | 3-$OCH_3$ | H | $SCH_3$ | |
| 1.83 | 2-$OCH_3$ | H | H | $SC(CH_3)_3$ | |
| 1.84 | 3-$OCH_3$ | H | H | $SCH(CH_3)_2$ | |
| 1.85 | 4-$OCH_3$ | H | H | $SCH(CH_3)_2$ | $n_D^{26}$ 1.6084 |
| 1.86 | 4-$C_6H_5$ | H | H | $SC(CH_3)_3$ | |
| 1.87 | 2-$C_6H_5$ | H | H | $SC(CH_3)_3$ | |
| 1.88 | 2-Cl | 4-Cl | H | $OCH_2CH{=}CH_2$ | $n_D^{40}$ 1.5830 |
| 1.89 | 4-Cl | 2-Cl | H | $SCH_2CH(CH_3)_2$ | Harz |
| 1.90 | 4-Cl | H | H | S-$C_6H_4CH_3$(4) | Smp. 155-156° |
| 1.91 | 4-$OCH_3$ | H | H | $SC(CH_3)_3$ | Smp. 56-59° |
| 1.92 | 4-Cl | 2-Cl | H | $SCH(C_2H_5)C_3H_7$(n) | $n_D^{48}$ 1.5810 |
| 1.93 | 4-Cl | 2-Cl | H | 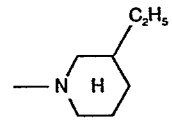 | $n_D^{48}$ 1.579 |
| 1.94 | 4-Cl | 2-Cl | H | O-$C_6H_4CH_3$(3) | Smp. 127-128° |
| 1.95 | 4-Cl | 2-Cl | H | $N(C_2H_5)_2$ | $n_D^{48}$ 1.5900 |
| 1.96 | 4-F | H | H | $OCH_2CF_3$ | Smp. 96-97° |
| 1.97 | 4-F | H | H | O-Cyclohexyl | $n_D^{23}$ 1.5638 |
| 1.98 | 4-F | H | H | $OCH_2CH_2CH_3$ | Smp. 60-61° |
| 1.99 | 4-Cl | 2-Cl | H | $OCH_2CH_2CH_3$ | Smp. 59-61° |
| 1.100 | 4-Cl | 2-Cl | H | $SC_2H_5$ | $n_D^{26}$ 1.6284 |
| 1.101 | 4-Cl | 2-Cl | H | $S(CH_2)_3CH_3$ | $n_D^{24}$ 1.6087 |
| 1.102 | 4-Cl | 2-Cl | H | $SCH_2CH_2N(CH_3)_2$ | |
| 1.103 | 4-Cl | 2-Cl | H | $SCH(CH_3)C_2H_5$ | $n_D^{27}$ 1.6075 |
| 1.104 | 4-Cl | 2-Cl | H | $SCH_2CH_2$-$C_6H_5$ | |
| 1.105 | 4-Cl | 2-Cl | H | 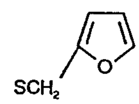 | |
| 1.106 | 4-Cl | 2-Cl | H | 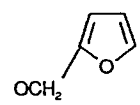 | |
| 1.107 | 4-Cl | 2-Cl | H | O-Cyclohexyl | |
| 1.108 | 4-Cl | 2-Cl | H | $NHC_4H_9$(n) | $n_D^{40}$ 1.5810 |
| 1.109 | 4-Cl | 2-Cl | H | $NHCH(CH_3)_2$ | |
| 1.110 | 4-Cl | 2-Cl | H | $NHC(CH_3)_3$ | Smp. 153° |
| 1.111 | 4-Cl | 2-Cl | H | NH-Cyclopropyl | |
| 1.112 | 4-Cl | 2-Cl | H | NH-Cyclohexyl | |
| 1.113 | 4-$CH_3$ | H | H | $OC(CH_3)_3$ | |
| 1.114 | 4-$CH_3$ | H | H | $SC(CH_3)_3$ | Smp. 101-102° |
| 1.115 | 4-$C(CH_3)_3$ | H | H | $SC(CH_3)_3$ | Smp. 161-162° |
| 1.116 | 4-$C(CH_3)_3$ | H | H | $OCH(CH_3)_2$ | |
| 1.117 | 4-$C(CH_3)_3$ | H | H | $SCH(C_2H_5)C_3H_7$(n) | $n_D^{25}$ 1.5674 |
| 1.118 | 4-$C(CH_3)_3$ | H | H | $OC(CH_3)_3$ | |
| 1.119 | 4-$C(CH_3)_3$ | H | H | $NHCH(CH_3)_2$ | |
| 1.120 | 4-$CF_3$ | H | H | $SC(CH_3)_3$ | Smp. 81-83° |
| 1.121 | 3-$OCH_3$ | 4-$OCH_3$ | 5-$OCH_3$ | $OC(CH_3)_3$ | |
| 1.122 | 3-$OCH_3$ | 4-$OCH_3$ | 5-$OCH_3$ | $SC_2H_5$ | |
| 1.123 | 3-$OCH_3$ | 4-$OCH_3$ | 5-$OCH_3$ | $SC(CH_3)_3$ | Smp. 123-124° |
| 1.124 | 3-$OCH_3$ | 4-$OCH_3$ | 5-$OCH_3$ | $SCH(C_2H_5)C_3H_7$(n) | Smp. 70° |
| 1.125 | 3-$N(CH_3)_2$ | H | H | $SC(CH_3)_3$ | |
| 1.126 | 3-$N(CH_3)_2$ | H | H | $OCH(CH_3)C_2H_5$ | |
| 1.127 | 2-J | H | H | $SC(CH_3)_3$ | Smp. 126-127° |
| 1.128 | 4-Br | H | H | $OCH(CH_3)_2$ | Smp. 95-96° |
| 1.129 | 2-J | H | H | $SC_3H_7$(iso) | $n_D^{27}$ 1.6390 |

| Verb. Nr. | $R_a$ | $R_b$ | $R_c$ | $R_1$ | Physik Konst. [°C] |
|---|---|---|---|---|---|
| 1.130 | 2-J | H | H | $SC_4H_9$(iso) | $n_D^{27}$ 1.6240 |
| 1.131 | 4-CN | H | H | $SC_4H_9$(tert.) | |
| 1.132 | 3-Cl | 5-Cl | H | $SC_3H_7$(iso) | |
| 1.133 | 3-Cl | 5-Cl | H | $OC_4H_9$(tert.) | |
| 1.134 | 3-Cl | 5-Cl | H | $SC_4H_9$(tert.) | |
| 1.135 | 3-Cl | 5-Cl | H | S-Cyclohexyl | |
| 1.136 | 3-Cl | 5-Cl | H | $OCH_2CF_3$ | |
| 1.137 | 4-F | H | H | $SCH(C_2H_5)C_3H_7(n)$ | $n_D^{28}$ 1.5710 |
| 1.138 | 4-Cl | H | H | $SC_2H_5$ | |
| 1.139 | 4-Cl | H | H | $SCH(C_2H_5)C_3H_7(n)$ | |
| 1.140 | 4-Cl | H | H | S-Cyclohexyl | |
| 1.141 | 4-Cl | H | H | $OC_4H_9$(tert.) | |
| 1.142 | 4-Cl | 2-Cl | H | $SC(CH_3)_2C_2H_5$ | Smp. 89° |
| 1.143 | 4-$CH_3$ | H | H | $SCH(CH_3)_2$ | Smp. 48-50° |
| 1.144 | 4-Cl | 2-Cl | H | $SCH_2CH_2CH(CH_3)_2$ | |
| 1.145 | 4-Cl | 2-Cl | H | $SCH_2CHOH$<br>\|<br>$CH_3$ | |
| 1.146 | 4-Cl | 2-Cl | H | $SCH_2CH_2OC_2H_5$ | |
| 1.147 | 4-Cl | 2-Cl | H | $SCH_2CH_2CN$ | |
| 1.148 | 4-Cl | 2-Cl | H | S-Cyclopentyl | |
| 1.149 | 4-Cl | 2-Cl | H | $SCH_2COOCH_3$ | |
| 1.150 | 4-Cl | 2-Cl | H | $SCH_2CH_2Si(OC_2H_5)_3$ | $n_D^{24}$ 1.5540 |
| 1.151 | 4-Cl | 2-Cl | H | $SCH_2CH_2CH_2Si(OCH_3)_3$ | |
| 1.152 | 4-Cl | 2-F | H | $SC(CH_3)_3$ | Smp. 101-103° |
| 1.153 | 4-Cl | 2-F | H | $SCH(CH_3)_2$ | Smp. 62-65° |
| 1.154 | 4-Cl | 2-F | H | $SC_3H_7(n)$ | |
| 1.155 | 4-Cl | 2-F | H | $SC_4H_9(n)$ | |
| 1.156 | 4-Cl | 2-F | H | S-Benzyl | Smp. 86-89° |
| 1.157 | 4-Cl | 2-F | H | S-$C_6H_4Cl(4)$ | $n_D^{40}$ 1.6386 |
| 1.158 | 4-Cl | 2-F | H | O-$C_6H_4Cl(4)$ | Smp. 111-123° |
| 1.159 | 4-Cl | 2-F | H | $NHC_4H_9$(sec) | Wachs |
| 1.160 | 4-Cl | 2-F | H | $OC_3H_7(n)$ | |
| 1.161 | 4-Cl | 2-F | H | $OC_3H_7$(iso) | |
| 1.162 | 4-Cl | 2-F | H | $SCH(C_2H_5)C_3H_7(n)$ | |
| 1.163 | 4-Cl | 2-F | H | $OC_4H_9$(tert.) | |
| 1.164 | 4-Cl | 2-Cl | H | $N(CH_3)_2$ | |
| 1.165 | 4-Cl | H | H | $N(CH_3)_2$ | |
| 1.166 | 4-Cl | H | H | 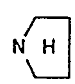 | |
| 1.167 | 4-Cl | H | H | 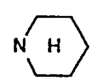 | |
| 1.168 | 4-Cl | H | H | 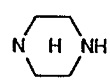 | |
| 1.69 | 4-Cl | H | H | 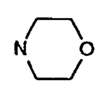 | |
| 1.170 | H | H | H | 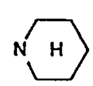 | |
| 1.171 | 4-Cl | H | H | 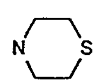 | |
| 1.172 | H | H | H | 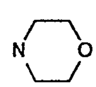 | |

| Verb. Nr. | $R_a$ | $R_b$ | $R_c$ | $R_1$ | Physik Konst. [°C] |
|---|---|---|---|---|---|
| 1.173 | 4-Cl | H | H | 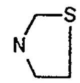 | |
| 1.174 | 2-Cl | 4-Cl | H | $OCH(CF_3)_2$ | |
| 1.175 | 4-$C_6H_5$ | H | H | $SCH_3$ | |
| 1.176 | 4-$C_6H_5$ | H | H | $SC_2H_5$ | |
| 1.177 | 4-$C_6H_5$ | H | H | $SC_3H_7$(n) | |
| 1.178 | 4-$C_6H_5$ | H | H | $SC_3H_7$(iso) | |
| 1.179 | 4-$C_6H_5$ | H | H | $SC_4H_9$(n) | |
| 1.180 | 4-$C_6H_5$ | H | H | $SC_4H_9$(iso) | |
| 1.181 | 4-$C_6H_5$ | H | H | $SC_4H_9$(sec) | |
| 1.182 | 4-$C_6H_5$ | H | H | $OCH_3$ | |
| 1.183 | 4-$C_6H_5$ | H | H | $OC_2H_5$ | |
| 1.184 | 4-$C_6H_5$ | H | H | $OC_3H_7$(n) | |
| 1.185 | 4-$C_6H_5$ | H | H | $OC_3H_7$(iso) | |
| 1.186 | 4-$C_6H_5$ | H | H | $OC_4H_9$(n) | |
| 1.187 | 4-$C_6H_5$ | H | H | $OC_4H_9$(iso) | |
| 1.188 | 4-$C_6H_5$ | H | H | $OC_4H_9$(sec) | |
| 1.189 | 4-$C_6H_5$ | H | H | $OC_4H_9$(tert.) | |
| 1.190 | 4-$C_6H_5$ | H | H | $OC_2CH{=}CH_2$ | |
| 1.191 | 4-$C_6H_5$ | H | H | $OCH_2C{\equiv}CH$ | |
| 1.192 | 4-$C_6H_5$ | H | H | $NHCH_3$ | |
| 1.193 | 4-$C_6H_5$ | H | H | $NHC_3H_7$(iso) | |
| 1.194 | 4-$C_6H_5$ | H | H | $N(C_2H_5)_2$ | |
| 1.195 | 4-$C_6H_5$ | H | H | 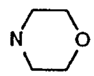 | |
| 1.196 | 2-Cl | H | H | $SC(CH_3)_3$ | |
| 1.197 | 2-Cl | H | H | $SC(CH_3)_2C_2H_5$ | |
| 1.198 | 2-Cl | H | H | $NHC(CH_3)_3$ | |
| 1.199 | 2-Cl | H | H | $OCH(CH_3)_2$ | |
| 1.200 | 2-Cl | H | H | $OC(CH_3)_3$ | |
| 1.201 | 2-Cl | H | H | $NHCH(CH_3)_2$ | |
| 1.202 | 2-Cl | H | H | $OC_3H_7$(n) | |
| 1.203 | 2-$CH_3$ | 4-$CH_3$ | H | $SCH(CH_3)_2$ | |
| 1.204 | 2-$CH_3$ | 4-$CH_3$ | H | $SC(CH_3)_2C_2H_5$ | |
| 1.205 | 2-$CH_3$ | 4-$CH_3$ | H | $OCH(CH_3)_2$ | |
| 1.206 | 2-$CH_3$ | 4-$CH_3$ | H | $NHCH(CH_3)_2$ | |
| 1.207 | 2-Cl | 5-Cl | H | $OCH(CH_3)C_2H_5$ | |
| 1.208 | 2-Cl | 5-Cl | H | $OCH(CH_3)_2$ | |
| 1.209 | 2-Cl | 5-Cl | H | $OC(CH_3)_3$ | |
| 1.210 | 2-Cl | 5-Cl | H | $SCH(CH_3)_2$ | |
| 1.211 | 2-Cl | 3-Cl | H | $OC_2H_5$ | |
| 1.212 | 2-Cl | 3-Cl | H | $OCH(CH_3)_2$ | |
| 1.213 | 2-Cl | 3-Cl | H | $NHCH(CH_3)_2$ | |
| 1.214 | 2-Cl | 3-Cl | H | $NHC(CH_3)_3$ | |
| 1.215 | 2-Cl | 3-Cl | H | $SC(CH_3)_3$ | |
| 1.216 | 2-Cl | 3-Cl | H | $SC(CH_3)_2C_2H_5$ | |
| 1.217 | 2-Cl | 3-Cl | H | $SCH(CH_3)C_2H_5$ | |
| 1.218 | 4-Cl | 2-F | H | $SCH_2CH{=}CH_2$ | $n_D^{40}$ 1.6107 |
| 1.219 | 4-Br | 2-Cl | H | $SC(CH_3)_3$ | Öl |
| 1.220 | 4-Br | 2-Cl | H | $SCH(CH_3)_2$ | Öl |
| 1.221 | 4-Br | 2-Cl | H | $SCH_2CH{=}CH_2$ | |
| 1.222 | 4-Br | 2-Cl | H | $SCH_2$-$C_6H_5$ | Öl |
| 1.223 | 4-Br | 2-Cl | H | S-$C_6H_4$(Cl) (4) | |
| 1.224 | 4-Br | 2-Cl | H | O-$C_6H_4$(Cl)(4) | Öl |
| 1.225 | 4-Br | 2-Cl | H | $C_3$<br>\|<br>$NHCHC_2H_5$ | |
| 1.226 | 4-Br | 2-Cl | H | $OC(CH_3)_3$ | Öl |

Tabelle 2: Verbindungen der Formel

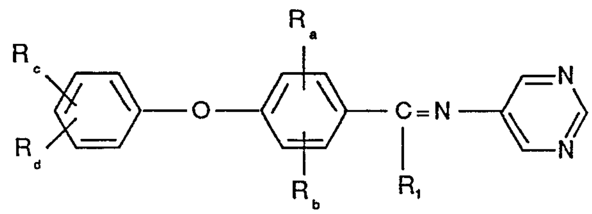

| Verb. Nr. | $R_a$ | $R_b$ | $R_c$ | $R_d$ | $R_1$ | Physik. Konst. [°C] |
|---|---|---|---|---|---|---|
| 2.1 | H | H | H | H | $SC(CH_3)_3$ | Smp. 94–96° |
| 2.2 | H | H | H | H | $SCH_3$ | |
| 2.3 | 2-Cl | H | H | H | $OCH(CH_3)_2$ | |
| 2.4 | 2-Cl | H | H | H | $SC(CH_3)_3$ | |
| 2.5 | 2-$CH_3$ | H | H | H | $OCH_2CF_3$ | |
| 2.6 | 2-$CH_3$ | H | H | H | $OCH_3$ | |
| 2.7 | H | H | 4-Cl | H | $SC(CH_3)_3$ | Smp. 111–114° |
| 2.8 | H | H | 4-Cl | H | $SCH(CH_3)_2$ | Öl |
| 2.9 | H | H | 4-$CH_3$ | H | $SCH_2CH_2CH_3$ | |
| 2.10 | H | H | 4-$CH_3$ | H | $OCH_2CF_3$ | |
| 2.11 | 2-Cl | H | 4-Cl | H | $OCH(CH_3)_2$ | Smp. 95° |
| 2.12 | 2-Cl | H | 4-Cl | H | $SC(CH_3)_2$ | Smp. 135–136° |
| 2.13 | 2-$CH_3$ | H | 4-$CH_3$ | H | $SCH(CH_3)_2$ | |
| 2.14 | 2-$CH_3$ | H | 4-$CH_3$ | H | $SCH_3$ | |
| 2.15 | 2-Cl | H | 4-$CH_3$ | H | $SC(CH_3)_3$ | |
| 2.16 | 2-Cl | H | 4-$CH_3$ | H | $OCH(CH_3)_2$ | |
| 2.17 | 2-$CH_3$ | H | 4-Cl | H | $OCH_3$ | |
| 2.18 | 2-$CH_3$ | H | 4-Cl | H | $SCH_2CH = CH_2$ | |
| 2.19 | 2-Cl | 3-Cl | 4-Cl | 2-Cl | S-Phenyl | |
| 2.20 | 2-Cl | 3-Cl | 4-$CH_3$ | H | $OCH_2C = CH$ | |
| 2.21 | 2-$CH_3$ | H | 4-Cl | 2-Cl | S-$C_6H_4F(4)$ | |
| 2.22 | H | H | H | 4-$CF_3$ | $SC(CH_3)_3$ | |
| 2.23 | H | H | H | 4-$OCF_3$ | $SCH(CH_3)_2$ | |
| 2.24 | H | H | H | H | $SCH(CH_3)_2$ | Öl |
| 2.25 | 2-Cl | H | 4-Cl | H | $SCH(CH_3)_2$ | Smp. 119–120° |
| 2.26 | 2-Cl | H | 4-Cl | H | $OC(CH_3)_3$ | |
| 2.27 | 2-Cl | H | 4-Cl | H | $NHCH(CH_3)_2$ | |
| 2.28 | 2-$CH_3$ | H | 4-$CH_3$ | H | $SC(CH_3)_3$ | |
| 2.29 | 2-Cl | H | 4-$CH_3$ | H | $SC(CH_3)_3$ | |
| 2.30 | 2-Cl | H | 4-$CH_3$ | H | $OCH(CH_3)_2$ | |
| 2.31 | 2-$CH_3$ | H | 4-Cl | H | $SCH(CH_3)_2$ | Smp. 109° |
| 2.32 | 2-Br | H | 4-Cl | H | $SC(CH_3)_3$ | |
| 2.33 | 2-Br | H | 4-Br | H | $OC(CH_3)_3$ | |
| 2.34 | 2-Br | H | 4-Br | H | $NHCH(CH_3)_2$ | |
| 2.35 | 2-Br | H | 4-Br | H | $SC(CH_3)_3$ | |
| 2.36 | 2-Br | H | 4-Br | H | S-Cyclohexyl | |
| 2.37 | 2-F | H | 4-Br | H | $SC(CH_3)_3$ | |
| 2.38 | 2-F | H | 4-Br | H | $OCH(CH_3)C_2H_5$ | |
| 2.39 | 2-F | H | 4-F | H | $SCH(CH_3)_2$ | |
| 2.40 | 2-$OCHF_2$ | H | 4-Br | H | $SC(CH_3)_3$ | |
| 2.41 | 2-$OCHF_2$ | H | 4-Cl | H | $SC(CH_3)_3$ | |
| 2.42 | 2-$CH_3$ | H | 4-Cl | H | $SC(CH_3)_3$ | Smp. 137–138° |
| 2.43 | 2-$CH_3$ | H | 4-Cl | H | $OC(CH_3)_3$ | |
| 2.44 | 2-$CH_3$ | H | 4-Cl | H | $SCH_2CH(CH_3)_2$ | |
| 2.45 | 2-$OCHF_2$ | H | 4-F | H | $SCH(CH_3)_2$ | |
| 2.46 | 2-Cl | H | 4-Br | H | $SC_4H_9$(tert.) | |
| 2.47 | 2-Cl | H | 4-Br | H | $SC_3H_7$(iso) | |
| 2.48 | 2-Cl | H | 4-Br | H | S-Benzyl | |
| 2.49 | 2-Cl | H | 4-Br | H | $OC_4H_9$(tert.) | |
| 2.50 | 2-Cl | H | 4-Cl | H | $SC(CH_3)_2C_2H_5$ | |
| 2.51 | 2-$CH_3$ | H | 4-Cl | H | $SC_4H_9$(sec.) | |
| 2.52 | H | H | H | H | $NHCH_3$ | |
| 2.53 | H | H | H | H | $NHC_3H_7$(iso) | |

| Verb. Nr. | $R_a$ | $R_b$ | $R_c$ | $R_d$ | $R_1$ | Physik. Konst. [°C] |
|---|---|---|---|---|---|---|
| 2.54 | H | H | H | H | $NHC_4H_9(n)$ | |
| 2.55 | H | H | H | H | $N(CH_3)_2$ | |
| 2.56 | H | H | H | H | $OCH_3$ | |
| 2.57 | H | H | H | H | $OC_3H_7(n)$ | |
| 2.58 | H | H | H | H | 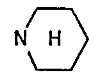 | |
| 2.59 | H | H | H | H | 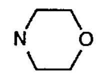 | |
| 2.60 | H | H | 4-Cl | H | $NHC_2H_5$ | |
| 2.61 | H | H | 4-Cl | H | $N(CH_3)C_2H_5$ | |
| 2.62 | H | H | 4-Cl | H | 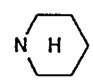 | |
| 2.63 | H | H | 4-Cl | H | 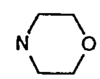 | |
| 2.64 | H | H | 4-Cl | H | 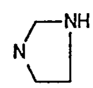 | |
| 2.65 | H | H | 3-Cl | 5-Cl | $N(C_2H_5)_2$ | |
| 2.66 | 2-Cl | H | 4-Cl | H | $NHC_3H_7(n)$ | |
| 2.67 | 2-Cl | H | 4-Cl | H | 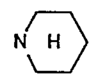 | |
| 2.68 | $2-CH_3$ | H | 4-Cl | H | 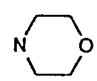 | |

Formulierungsbeispiele (% = Gewichtsprozent)

| F1. Emulsions-Konzentrate | a) | b) | c) |
|---|---|---|---|
| Wirkstoff aus den Tabellen | 25% | 40% | 50% |
| Ca-Dodecylbenzolsulfonat | 5% | 8% | 6% |
| Ricinusöl-polyethylenglykolether (36 Mol Ethylenoxid) | 5% | - | - |
| Tributhylphenoyl-polyethylenglykolether (30 Mol Ethylenoxid) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden.

| F2. Granulate | a) | b) |
|---|---|---|
| Wirkstoff aus den Tabellen | 5% | 10% |
| Kaolin | 94% | - |
| Hochdisperse Kieselsäure | 1% | - |
| Attapulgit | - | 90% |

| F3. Spritzpulver | a) | b) | c) |
|---|---|---|---|
| Wirkstoff aus den Tabellen | 25% | 50% | 75% |
| Na-Ligninsulfonat | 5% | 5% | - |
| Na-Laurylsulfat | 3% | - | 5% |
| Na-Diisobuthylnaphthalinsulfonat | - | 6% | 10% |
| Octylphenolpolyethylenglykolether (7–8 Mol Ethylenoxid) | - | 2% | - |
| Hochdisperse Kieselsäure | 5% | 10% | 10% |
| Kaolin | 62% | 27% | — |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| F4. Stäubemittel | a) | b) |
|---|---|---|
| Wirkstoff aus den Tabellen | 5% | 8% |
| Talkum | 95% | - |
| Kaolin | - | 92% |

Man erhält anwendungsfertige Stäubemittel, indem der Wirkstoff mit dem Träger vermischt und auf einer geeigneten Mühle vermahlen wird.

Biologische Beispiele

Beispiel B1

Wirkung gegen Puccinia graminis auf Weizen

a) Residual-protektive Wirkung

Weizenpflanzen wurden 6 Tage nach der Aussaat mit einer aus Spritzpulver des Wirkstoffes hergestellten Spritzbrühe (0,006% Aktivsubstanz) besprüht. Nach 24 Stunden wurden die behandelten Pflanzen mit einer Uredosporensuspensionen des Pilzes infiziert. Nach einer Inkubation während 48 Stunden bei 95-100% relativer Luftfeuchtigkeit und ca. 20°C wurden die infizierten Pflanzen in einem Gewächshaus bei ca. 22°C aufgestellt. Die Beurteilung der Rostpustelnentwicklung erfolgte 12 Tage nach der Infektion.

b) Systemische Wirkung

Zu Weizenpflanzen wurde 5 Tage nach der Aussaat eine aus Spritzpulver des Wirkstoffs hergestellte Spritzbrühe gegossen (0,002% Aktivsubstanz bezogen auf das Bodenvolumen). Nach 48 Stunden wurden die behandelten Pflanzen mit einer Uredosporensuspension des Pilzes infiziert. Nach einer Inkubation während 48 Stunden bei 95-100% relativer Luftfeuchtigkeit und ca. 20°C wurden die infizierten Pflanzen in einem Gewächshaus bei ca. 22°C aufgestellt. Die Beurteilung der Rostpustelnentwicklung erfolgte 12 Tage nach der Infektion.

Verbindungen aus den Tabellen zeigten gegen Puccinia-Pilze eine gute Wirkung. Unbehandelte aber infizierte Kontrollpflanzen zeigten einen Puccinia-Befall von 100%. Unter anderen hemmten die Verbindungen Nr. 1.1.-1.4, 1.8, 1.12-1.14, 1.17-1.24, 1.41, 1.88, 1.89, 1.92, 1.95, 1.99-1.111, 1.117, 1.137-1.165, 1.168-1.178, 1.185, 1.187, 1.196-1.226, 2.7, 2.8, 2.11, 2.12, 2.15-2.18, 2.24-2.26, 2.47, 2.57, 75 und 76 den Puccinia-Befall auf 0 bis 5%.

Beispiel B2

Wirkung gegen Cercospora arachidicola auf Erdnusspflanzen

a) Residual-protektive Wirkung

10-15 cm hohe Erdnusspflanzen wurden mit einer aus Spritzpulver der Wirksubstanz hergestellten Spritzbrühe (0,002% Aktivsubstanz) besprüht und 48 Stunden später mit einer Konidiensuspension des Pilzes infiziert. Die infizierten Pflanzen wurden währen 72 Stunden bei ca. 21°C und hoher Luftfeuchtigkeit inkubiert und anschliessend bis zum Auftreten der typischen Blattflecken in einem Gewächshaus aufgestellt. Die Beurteilung der fungiziden Wirkung erfolgte 12 Tage nach der Infektion basierend auf Anzahl und Grösse der auftretenden Flecken.

b) Systemische Wirkung

Zu 10-15 cm hohen Erdnusspflanzen wurde eine aus Spritzpulver des Wirkstoffes hergestellte Spritzbrühe gegossen (0,06% Aktivsubstanz bezogen auf das Erdvolumen). Nach 48 Stunden wurden die behandelten Pflanzen mit einer Konidiensuspension des Pilzes infiziert und 72 Stunden bei ca. 21°C und hoher Luftfeuchtigkeit inkubiert. Anschliessend wurden die Pflanzen im Gewächshaus aufgestellt und nach 11 Tagen der Pilzbefall beurteilt.

Im Vergleich zu unbehandelten, aber infizierten Kontrollpflanzen (Anzahl und Grösse der Flecken = 100%), zeigten Erdnusspflanzen, die mit Wirkstoffen aus den Tabellen behandelt wurden, einen stark reduzierten Cercospora-Befall. So verhinderten die Verbindungen Nr. 1.1, 1.3, 1.4, 1.12, 1.23, 1.30, 1.45, 1.99, 2.11, 2.12, 2.15, 2.16, 75 und 76 in obigen Versuchen das Auftreten von Flecken fast vollständig (0-10%).

Beispiel B3

Wirkung gegen Erysiphe graminis auf Gerste

a) Residual-protektive Wirkung

Ca. 8 cm hohe Gerstenpflanzen wurden mit einer aus Spritzpulver des Wirkstoffes hergestellten Spritzbrühe (0,006 Aktivsubstanz) besprüht. Nach 3-4 Stunden wurden die behandelten Pflanzen mit Konidien des Pilzes bestäubt. Die infizierten Gerstenpflanzen wurden in einem Gewächshaus bei ca. 22°C aufgestellt und der Pilzbefall nach 10 Tagen beurteilt.

b) Systemische Wirkung

Zu ca. 8 cm hohen Gerstenpflanzen wurde eine aus Spritzpulver des Wirkstoffes hergestellte Spritzbrühe gegossen (0,002% Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit Konidien des Pilzes bestäubt. Die infizierten Gerstenpflanzen wurden in einem Gewächshaus bei ca. 22°C aufgestellt und der Pilzbefall nach 10 Tagen beurteilt.

Verbindungen der Formel I zeigten eine gute Wirkung gegen Erysiphe-Pilze. Unbehandelte, aber infizierte Kontrollpflanzen zeigten einen Erysiphe-Befall von 100%. Unter anderen Verbindungen aus den Tabellen hemmten die Verbindungen Nr. 1.1-1.5, 1.7-1.30, 1.45, 1.60, 1.70, 1.88-1.194, 2.1, 2.7, 2.8, 2.11, 2.12, 2.15, 2.16, 2.24, 2.25, 2.31, 2.42, 75, 76, 99, 108e und 114 den Pilzbefall bei Gerste auf 0 bis 5%.

Beispiel B4

Residual-protektive Wirkung gegen Venturia inaequalis auf Apfeltrieben.

Apfelstecklinge mit 10-20 cm langen Frischtrieben wurden mit einer aus Spritzpulver des Wirkstoffes hergestellten Spritzbrühe (0,06% Aktivsubstanz) besprüht. Nach 24 Stunden wurden die behandelten Pflanzen mit einer Konidiensuspension des Pilzes infiziert. Die Pflanzen wurden dann während 5 Tagen bei 90-100% relativer Luftfeuchtigkeit inkubiert und während 10 weiteren Tagen in einem Gewächshaus bei 20-24°C aufgestellt. Der Schorfbefall wurde 15 Tage nach der Infektion beurteilt. Verbindungen aus den Tabellen 1 und 2, besonders Verb. 1.3 und 1.12 bewirkten eine deutliche Hemmung des Krankheitsbefalls. Unbehandelte aber infizierte Triebe zeigten einen 100%igen Venturia-Befall.

Beispiel B5

Wirkung gegen Botrytis cinerea auf Bohnen. Residual protektive Wirkung

Ca. 10 cm hohe Bohnen-Pflanzen wurden mit einer aus Spritzpulver des Wirkstoffes hergestellten Spritzbrühe (0,02% Aktivsubstanz) besprüht. Nach 48 Stunden wurden die behandelten Pflanzen mit einer Konidiensuspension des Pilzes infiziert. Nach einer Inkubation der infizierten Pflanzen während 3 Tagen bei 95–100% relativer Luftfeuchtigkeit und 21 °C erfolgte die Beurteilung des Pilzbefalls. Die Verbindungen aus den Tabellen 1 und 2 hemmten in vielen Fällen die Pilzinfektion sehr stark. Bei einer Konzentration von 0,02% erwiesen sich z. B. die Verbindungen Nr. 1.1, 1.3, 1.4, 1.12, 2.11, 2.12, 2.15 und 2.16 (Krankheitsbefall 0 bis 5%). Der Botrytis-Befall unbehandelter aber infizierter Bohnenpflanzen betrug 100%.

**Patentansprüche**

1. Verbindungen der Formel I

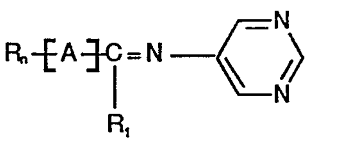

(I)

worin
A für Phenyl, Naphthyl, Biphenyl, Phenoxyphenyl oder Phenylthiophenyl steht; R Halogen, Cyano, Nitro, $C_1$–$C_4$-Alkyl, $C_1$–$C_4$-Alkoxycarbonyl, Tris($C_1$–$C_4$-Alkoxy)silyl, Hydroxy, $C_1$–$C_4$-Alkoxy, $C_1$–$C_4$-Haloalkoxy, $C_1$–$C_4$-Haloalkyl oderDi($C_1$–$C_4$-alkyl)amino bedeutet; n für 0, 1, 2, 3, 4 oder 5 steht; $R_1$ eine der Gruppen $OR_2$, $SR_2$ oder $N(R_3)(R_4)$ repräsentiert, wobei $R_2$ für $C_1$–$C_8$-Alkyl, $C_3$–$C_8$-Alkenyl, $C_3$–$C_8$-Alkinyl oder für einen durch R einfach substituierten $C_1$–$C_8$-Alkylrest oder für einen unsubstituierten oder ein- bis dreifach durch R substituierten Rest aus der Reihe Phenyl, Phenalkyl($C_1$–$C_4$), $C_3$–$C_7$-Cycloalkyl oder Furfuryl steht; und $R_3$ und $R_4$ unabhängig voneinander für Wasserstoff oder $C_1$–$C_4$-Alkyl stehen oder gemeinsam mit dem Aminstickstoff einen gesättigten fünf- oder sechs-gliedrigen Heterocyclus bilden, der als Heteroatom entweder nur den Aminstickstoff oder ein weiteres Heteroatom N, O oder S enthält.

2. Verbindungen der Formel I nach Anspruch 1, worin A für Phenyl, Naphthyl, Biphenyl, Phenoxyphenyl oder Phenylthiophenyl steht; R Halogen, Cyano, Nitro, $C_1$–$C_4$-Alkyl, $C_1$–$C_4$-Alkoxy, $C_1$–$C_4$-Haloalkoxy, $C_1$–$C_4$-Haloalkyl oderDi($C_1$–$C_4$-alkyl)amino bedeutet; n für 0, 1, 2, 3, 4 oder 5 steht; $R_1$ eine der Gruppen $OR_2$, $SR_2$ oder $N(R_3)(R_4)$ repräsentiert, wobei $R_2$ für $C_1$–$C_8$-Alkyl, $C_3$–$C_8$-Alkenyl, $C_3$–$C_8$-Alkinyl oder für einen unsubstituierten oder ein- bis dreifach durch R substituierten Rest aus der Reihe Phenyl, Benzyl, $C_3$–$C_7$-Cycloalkyl oder Furfuryl steht; und $R_3$ und $R_4$ unabhängig voneinander für Wasserstoff oder $C_1$–$C_4$-Alkyl stehen oder gemeinsam mit dem Aminstickstoff einen gesättigten fünf- oder sechs-gliedrigen Heterocyclus bilden, der als Heteroatom entweder nur den Aminstickstoff oder ein weiteres Heteroatom enthält.

3. Verbindungen der Formel I nach Anspruch 1, worin A für Phenyl steht; R Hydroxy, Halogen, Cyano, Methyl, Ethyl, Methoxycarbonyl, Ethoxycarbonyl, Methoxy, Ethoxy, $CF_3$, Trimethoxysilyl, $C_1$–$C_2$-Haloalkoxy, $N(CH_3)_2$ oder $N(C_2H_5)_2$ bedeutet; n für 1, 2 oder 3 steht; $R_1$ eine der Gruppen $OR_2$ oder $SR_2$ repräsentiert; wobei $R_2$ für $C_1$–$C_8$-Alkyl, $C_3$–$C_8$-Alkenyl, $C_3$–$C_8$-Alkinyl oder für einen durch Hydroxy, Halogen, Cyano, Methoxycarbonyl, Ethoxycarbonyl, Methoxy, Ethoxy, Trimethoxysilyl, Triethoxysilyl, $N(CH_3)_2$ oder $N(C_2H_5)_2$ einfach substituierten $C_1$–$C_4$-Alkylrest oder für einen unsubstituierten oder ein- bis dreifach durch Halogen, Cyano, Methyl, Ethyl, Methoxy, Ethoxy, $CF_3$, $C_1$–$C_2$-Haloalkoxy oder $N(C_1$–$C_2$-Alkyl$)_2$ substituierten Rest aus der Reihe Phenyl, Phenethyl, Benzyl, $C_5$–$C_6$-Cycloalkyl und Furfuryl steht.

4. Verbindungen der Formel I nach Anspruch 1, dadurch gekennzeichnet, dass A für Phenyl steht; R Halogen, Cyano, Methyl, Ethyl, Methoxy, Ethoxy, $CF_3$, $C_1$–$C_2$-Haloalkoxy, $N(CH_3)_2$oder $N(C_2H_5)_2$ bedeutet; n für 1, 2 oder 3 steht; $R_1$ eine der Gruppen $OR_2$ oder $SR_2$ repräsentiert; wobei $R_2$ für $C_1$–$C_8$-Alkyl, $C_3$–$C_8$-Alkenyl, $C_3$–$C_8$-Alkinyl oder für einen unsubstituierten oder ein- bis dreifach durch Halogen, Cyano, Methyl, Ethyl, Methoxy, Ethoxy, $CF_3$, $C_1$–$C_2$-Haloalkoxy oder $N(C_1$–$C_2$-Alkyl$)_2$ substituierten Rest aus der Reihe Phenyl, Benzyl, $C_5$–$C_6$-Cycloalkyl und Furfuryl steht.

5. Verbindungen der Formel I nach Anspruch 4, dadurch gekennzeichnet, dass A für Phenyl steht; R Fluor, Chlor, Brom, Methyl, Methoxy oder $CF_3$ bedeutet; n für 1 oder 2 steht; $R_1$ für $OR_2$ oder $SR_2$ steht; wobei $R_2$$C_1$–$C_5$-Alkyl, $C_3$–$C_4$-Alkenyl, $C_3$–$C_4$-Alkinyl, Cyclopentyl, Cyclohexyl, Furfuryl oder einen unsubstituierten oder ein- bis dreifach durch Fluor, Chlor, Brom, Methyl, Methoxy oder $CF_3$ substituierten Rest aus der Reihe Phenyl und Benzyl bedeutet.

6. Verbindungen der Formel I nach Anspruch 5, dadurch gekennzeichnet, dass n für 2 steht, R für Fluor oder Chlor steht; die an den 2- und 4-Positionen des Phenylrings substituiert sind; $R_1$ für $OR_2$ oder $SR_2$ steht; wobei $R_2$ $C_1$–$C_5$-Alkyl, Allyl, Propargyl, Cyclohexyl, Benzyl oder unsubstituiertes oder ein- bis dreifach durch Fluor, Chlor, Brom, Methyl, Methoxy oder $CF_3$ substituiertes Phenyl bedeutet.

7. Verbindungen der Formel I nach Anspruch 1, worin A für Phenoxyphenyl und n für 0, 1, 2, 3, 4 oder 5 steht, während die übrigen Substituenten wie in Anspruch 3 definiert sind.

8. Verbindungen der Formel I nach Anspruch 1, dadurch gekennzeichnet, dass A für Phenoxyphenyl steht; R Halogen, Cyano, Methyl, Ethyl, Methoxy, Ethoxy, $CF_3$, $C_1$–$C_2$-Haloalkoxy, $N(CH_3)_2$ oder $N(C_2H_5)_2$ bedeutet, n für 0, 1, 2, 3, 4 oder 5 steht; $R_1$ eine der Gruppen $OR_2$ oder $SR_2$ repräsentiert; wobei $R_2$ für $C_1$–$C_8$-Alkyl, $C_3$–$C_8$-Alkenyl, $C_3$–$C_8$-Alkinyl oder für einen unsubstituierten oder ein- bis dreifach durch Halogen, Cyano, Methyl, Ethyl, Methoxy, Ethoxy, $CF_3$, $C_1$–$C_2$-Haloalkoxy oder $N(C_1$–$C_2$-Alkyl$)_2$ substituierten Rest aus der Reihe Phenyl, Benzyl, $C_5$–$C_6$-Cycloalkyl oder Furfuryl steht.

9. Verbindungen der Formel I nach Anspruch 8, dadurch gekennzeichnet, dass A für (p-Phenoxy)-phenyl steht.

10. Verbindungen der Formel I nach Anspruch 9, dadurch gekennzeichnet, dass A für (p-Phenoxy)phenyl steht; R Fluor, Chlor, Brom, Methyl, Methoxy oder $CF_3$ bedeutet; n für 0, 1, 2, 3 oder 4

steht; $R_1$ für $OR_2$ oder $SR_2$ steht; wobei$R_2$ $C_1$-$C_5$-Alkyl, $C_3$-$C_4$-Alkenyl, $C_3$-$C_4$-Alkinyl, Cyclopentyl, Cyclohexyl, Furfuryl oder einen unsubstituierten oder ein- bis dreifach durch Fluor, Chlor, Brom, Methyl, Methoxy oder $CF_3$ substituierten Rest aus der Reihe Phenyl und Benzyl bedeutet.

11. Verbindungen der Formel I nach Anspruch 10, dadurch gekennzeichnet, dass A für (p-Phenoxy)phenyl steht; R Fluor, Chlor, Methyl, Methoxy oder $CF_3$ bedeutet; n für 0, 1, 2 oder 3 steht; $R_1$ für $OR_2$ oder $SR_2$ steht; wobei $R_2$ für $C_1$-$C_5$-Alkyl, Allyl, Propargyl, Cyclohexyl, Benzyl oder unsubstituiertes oder ein- bis dreifach durch Fluor, Chlor, Brom, Methyl, Methoxy oder $CF_3$ substituiertes Phenyl steht.

12. Verbindungen der Formel I nach Anspruch 1, worin A für Phenyl oder p-Phenoxyphenyl steht; n 0, 1, 2 oder 3 bedeutet, R Halogen, Cyano, Methyl, Ethyl, Methoxy, Ethoxy, Methoxycarbonyl, Ethoxycarbonyl, $CF_3$ oder $C_1$-$C_2$-Haloalkoxy darstellt, $R_1$ die Gruppe $N(R_3)(R_4)$ bedeutet und $R_3$ und $R_4$ unabhängig voneinander für Wasserstoff oder $C_1$-$C_4$-Alkyl stehen oder gemeinsam mit dem Aminstickstoff einen Pyrrolidin-, Piperidin-, Piperazin- oder Morpholin-Ring bilden.

13. Verbindungen der Formel I nach Anspruch 12, worin R Fluor, Chlor oder Methyl darstellt.

14. Verbindungen der Formel I nach Anspruch 1, worin A für p-Biphenyl steht, n = 0 ist, $R_1$ entweder $OR_2$ oder $SR_2$ bedeutet und $R_2$ $C_1$-$C_4$-Alkyl, Allyl oder Propargyl ist.

15. Eine Verbindung der Formel I nach Anspruch 1, dadurch gekennzeichnet, dass sie ausgewählt ist aus der Reihe:

5-[2,4-Dichlorbenzyl-C-(tert.butylthio)imino]pyrimidin (Nr. 1.1);
5-[4-(p-Chlorphenoxy)-2-methylbenzyl- C-(tert. butylthio)imino]-pyrimidin (Nr. 2.15);
5-[4-(p-Chlorphenoxy)-2-methylbenzyl-C-(isopropoxy)imino]pyrimidin (Nr. 2.16);
5-[4-(p-Chlorphenoxy)-2-chlorbenzyl-C-(tert.butylthio)imino]-pyrimidin (Nr. 2.12);
5-[4-(p-Chlorphenoxy)-2-chlorbenzyl-C-(isopropoxy)imino]pyrimidin (Nr. 2.11).
5-[2,4-Dichlorbenzyl-C-(isopropoxy)imino]pyrimidin (Nr. 1.12)
5-[2,4-Dichlorbenzyl-C-(tert.butoxy)imino]pyrimidin (Nr. 1.13)
5-[2,4-Dichlorbenzyl-C-(allyloxy)imino]pyrimidin (Nr. 1.88)
5-[2,4-Dichlorbenzyl-C-(n-propoxy)imino]pyrimidin (Nr. 1.99)
5-[4-Chlorbenzyl-C-(tert.butylthio)imino]pyrimidin (Nr. 1.17)
5-[2,4-Dichlorbenzyl-C-(trifluorethoxy)imino]pyrimidin (Nr. 1.14)
5-[2,4-Dichlorbenzyl-C-(diethylamino)imino]pyrimidin (Nr. 1.95)
5-[2,4-Dichlorbenzyl-C-(tert.butylamino)imino]pyrimidin (Nr. 1.110)

16. Verfahren zur Herstellung von Verbindungen der Formel I gemäss Anspruch 1, dadurch gekennzeichnet, dass man eine Verbindung der Formel II

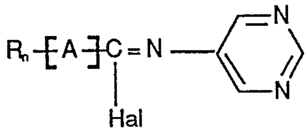 (II)

in Gegenwart einer Base mit einer Verbindung der Formel III

$R_1$-H (III)

umsetzt; dabei haben $R_n$, A und $R_1$ in den Formeln II und III die unter Formel I angegebenen Bedeutungen, Hal steht für Halogen, vorzugsweise für Chlor oder Brom.

17. Schädlingsbekämpfungsmittel zur Bekämpfung oder Verhütung eines Befalls durch Mikroorganismen, dadurch gekennzeichnet, dass es als mindestens eine aktive Komponente eine Verbindung der Formel I gemäss Anspruch 1 enthält.

18. Mittel gemäss Anspruch 17, dadurch gekennzeichnet, dass es als mindestens eine aktive Komponente eine Verbindung der Formel I gemäss Anspruch 2 enthält.

19. Mittel gemäss Anspruch 17, dadurch gekennzeichnet, dass es als mindestens eine aktive Komponente eine Verbindung der Formel I gemäss Anspruch 3 enthält.

20. Mittel gemäss Anspruch 17, dadurch gekennzeichnet, dass es als mindestens eine aktive Komponente eine Verbindung der Formel I gemäss einem der Ansprüche 4 bis 15 enthält.

21. Verfahren zur Bekämpfung oder Verhütung eines Befalls von Kulturpflanzen durch phytopathogene Mikroorganismen, dadurch gekennzeichnet, dass man eine Verbindung der Formel I auf die Pflanze oder deren Standort appliziert.

22. Verwendung von Verbindungen der Formel I zur Bekämpfung von Pflanzenkrankheiten, die auf Mikroorganismen zurückzuführen sind.

23. Verwendung gemäss Anspruch 22 von Verbindungen der Formel I gemäss einem der Ansprüche 2 bis 15.

24. Verwendung gemäss Anspruch 22, dadurch gekennzeichnet, dass es sich bei den Mikroorganismen um phytopathogene Pilze handelt.

25. Verbindungen der Formel II

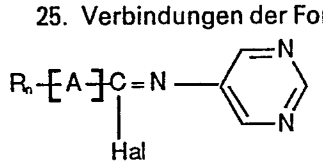 (II)

worin $R_n$ und A die unter Formel I nach Anspruch 1 angegebenen Bedeutungen haben und Hal für Halogen, vorzugsweise Chlor oder Brom steht.

## Claims

1. A compound of the formula I

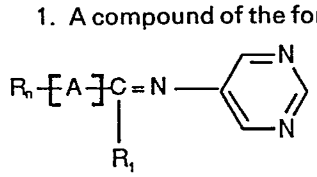 (I)

wherein
A is phenyl, naphthyl, biphenyl, phenoxyphenyl or phenylthiophenyl, R is halogen, cyano, nitro, $C_1$–$C_4$-alkyl, $C_1$–$C_4$-alkoxy-carbonyl, tris($C_1$–$C_4$-alkoxy)silyl, hydroxyl, $C_1$–$C_4$-alkoxy, $C_1$–$C_4$-haloalkoxy, $C_1$–$C_4$-haloalkyl or di($C_1$–$C_4$-alkyl)amino, n is 0, 1, 2, 3, 4 or 5, $R_1$ is one of the groups $OR_2$, $SR_2$ or $N(R_3)(R_4)$, in which $R_2$ is $C_1$–$C_8$-alkyl, $C_3$–$C_8$-alkenyl, $C_3$–$C_8$-alkynyl, or a $C_1$–$C_8$-alkyl group monosubstituted by R, or a radical from the group comprising phenyl, phenalkyl($C_1$–$C_4$), $C_3$–$C_7$-cycloalkyl and furfuryl, which is unsubstituted or mono- to trisubstituted by R, and $R_3$ and $R_4$ independently of one another are each hydrogen or $C_1$–$C_4$-alkyl, or together with the amine nitrogen form a saturated, five- or six-membered heterocycle which contains as hetero atom either just the amine nitrogen or a further hetero atom N, O or S.

2. A compound of the formula I according to claim 1, wherein A is phenyl, naphthyl, biphenyl, phenoxyphenyl or phenylthiophenyl, R is halogen, cyano, nitro, $C_1$–$C_4$-alkyl, $C_1$–$C_4$-alkoxy, $C_1$–$C_4$-haloalkoxy, $C_1$–$C_4$-haloalkyl or di($C_1$–$C_4$-alkyl)amino, n is 0, 1, 2, 3, 4 or 5, $R_1$ is one of the groups $OR_2$, $SR_2$ or $N(R_3)$ $(R_4)$, in which $R_2$ is $C_1$–$C_8$-alkyl, $C_3$–$C_8$-alkenyl, $C_3$–$C_8$-alkynyl, or a radical from the group comprising phenyl, benzyl, $C_3$–$C_7$-cycloalkyl and furfuryl, which is unsubstituted or mono- to trisubstituted by R, and $R_3$ and $R_4$ independently of one another are each hydrogen or $C_1$–$C_4$-alkyl, or together with the amine nitrogen form a saturated, five- or six-membered heterocycle containing as hetero atom either just the amine nitrogen or a further hetero atom.

3. A compound of the formula I according to claim 1, wherein A is phenyl, R is hydroxyl, halogen, cyano, methyl, ethyl, methoxycarbonyl, ethoxycarbonyl, methoxy, ethoxy, $CF_3$, trimethoxysilyl, $C_1$–$C_2$-haloalkoxy, $N(CH_3)_2$ or $N(C_2H_5)_2$, n is 1, 2 or 3, $R_1$ is one of the groups $OR_2$ or $SR_2$, in which $R_2$ is $C_1$–$C_8$-alkyl, $C_3$–$C_8$-alkenyl, $C_3$–$C_8$-alkynyl, or a $C_1$–$C_4$-alkyl group which is monosubstituted by hydroxyl, halogen, cyano, methoxycarbonyl, ethoxycarbonyl, methoxy, ethoxy, trimethoxysilyl, triethoxysilyl, $N(CH_3)_2$ or $N(C_2H_5)_2$, or is a radical from the group comprising phenyl, phenethyl, benzyl, $C_5$–$C_6$-cycloalkyl and furfuryl, which is unsubstituted or mono- to trisubstituted by halogen, cyano, methyl, ethyl, methoxy, ethoxy, $CF_3$, $C_1$–$C_2$-haloalkoxy or N($C_1$–$C_2$-alkyl)$_2$.

4. A compound of the formula I according to claim 1, wherein A is phenyl, R is halogen, cyano, methyl, ethyl, methoxy, ethoxy, $CF_3$, $C_1$–$C_2$-haloalkoxy, $N(CH_3)_2$ or $N(C_2H_5)_2$, n is 1, 2 or 3, $R_1$ is one of the groups $OR_2$ or $SR_2$, in which $R_2$ is $C_1$–$C_8$-alkyl, $C_3$–$C_8$-alkenyl, $C_3$–$C_8$-alkynyl, or a radical from the group comprising phenyl, benzyl, $C_5$–$C_6$-cycloalkyl and furfuryl, which is unsubstituted or mono- to trisubstituted by halogen, cyano, methyl, ethyl, methoxy, ethoxy, $CF_3$, $C_1$–$C_2$-haloalkoxy or N($C_1$–$C_2$-alkyl)$_2$.

5. A compound of the formula I according to claim 4, wherein A is phenyl, R is fluorine, chlorine, bromine, methyl, methoxy or $CF_3$, n is 1 or 2, $R_1$ is $OR_2$ or $SR_2$, in which $R_2$ is $C_1$–$C_5$-alkyl, $C_3$–$C_4$-alkenyl, $C_3$–$C_4$-alkynyl, cyclopentyl, cyclohexyl or furfuryl, or is a radical from the group comprising phenyl and benzyl, which is unsubstituted or mono- to trisubstituted by fluorine, chlorine, bromine, methyl, methoxy or $CF_3$.

6. A compound of the formula I according to claim 5, wherein n is 2, R is fluorine or chlorine, in the 2- and 4-positions of the phenyl ring, $R_1$ is $OR_2$ or $SR_2$, in which $R_2$ is $C_1$–$C_5$-alkyl, allyl, propargyl, cyclohexyl or benzyl, or phenyl which is unsubstituted or mono- to trisubstituted by fluorine, chlorine, bromine, methyl, methoxy or $CF_3$.

7. A compound of the formula I according to claim 1, wherein A is phenoxyphenyl, and n is 0, 1, 2, 3, 4 or 5, whilst the remaining substituents are as defined in claim 3.

8. A compound of the formula I according to claim 1, wherein A is phenoxyphenyl, R is halogen, cyano, methyl, ethyl, methoxy, ethoxy, $CF_3$, $C_1$–$C_2$-haloalkoxy, $N(CH_3)_2$ or $n(C_2H_5)_2$, n is 0, 1, 2, 3, 4 or 5, $R_1$ is one of the groups $OR_2$ or $SR_2$, in which $R_2$ is $C_1$–$C_8$-alkyl, $C_3$–$C_8$-alkenyl, $C_3$–$C_8$-alkynyl, or a radical from the group comprising phenyl, benzyl, $C_5$–$C_6$-cycloalkyl and furfuryl, which is unsubstituted or mono- to trisubstituted by halogen, cyano, methyl, ethyl, methoxy, ethoxy, $CF_3$, $C_1$–$C_2$-haloalkoxy or N($C_1$–$C_2$-alkyl)$_2$.

9. A compound of the formula I according to claim 8, wherein A is (p-phenoxy)-phenyl.

10. A compound of the formula I according to claim 9, wherein A is (p-phenoxy)phenyl, R is fluorine, chlorine, bromine, methyl, methoxy or $CF_3$, n is 0, 1, 2, 3 or 4, $R_1$ is $OR_2$ or $SR_2$, in which $R_2$ is $C_1$–$C_5$-alkyl, $C_3$–$C_4$-alkenyl, $C_3$–$C_4$-alkynyl, cyclopentyl, cyclohexyl, furfuryl, or a radical from the group comprising phenyl and benzyl, which is unsubstituted or mono- to trisubstituted by fluorine, chlorine, bromine, methyl, methoxy or $CF_3$.

11. A compound of the formula I according to claim 10, wherein A is (p-phenoxy)phenyl, R is fluorine, chlorine, methyl, methoxy or $CF_3$, n is 0, 1, 2 or 3, $R_1$ is $OR_2$ or $SR_2$, in which $R_2$ is $C_1$–$C_5$-alkyl, allyl, propargyl, cyclohexyl, benzyl, or phenyl which is unsubstituted or mono- to trisubstituted by fluorine, chlorine, bromine, methyl, methoxy or $CF_3$.

12. A compound of the formula I according to claim 1, wherein A is phenyl or p-phenoxyphenyl, n is 0, 1, 2 or 3, R is halogen, cyano, methyl, ethyl, methoxy, ethoxy, methoxycarbonyl, ethoxycarbonyl, $CF_3$ or $C_1$–$C_2$-haloalkoxy, $R_1$ is the group $N(R_3)(R_4)$, and $R_3$ and $R_4$ independently of one another are each hydrogen or $C_1$–$C_4$-alkyl, or together with the amine nitrogen form a pyrrolidine, piperidine, piperazine or morpholine ring.

13. A compound of the formula I according to claim 12, wherein R is fluorine, chlorine or methyl.

14. A compound of the formula I according to claim 1, wherein A is p-biphenyl, n is 0, $R_1$ is either $OR_2$ or $SR_2$, and $R_2$ is $C_1$–$C_4$-alkyl, allyl or propargyl.

15. A compound of the formula I according to

claim 1, which is selected from the series comprising:

5-[2,4-dichlorobenzyl-C-(tert-butylthio)imino] pyrimidine (No 1.1),
5-[4-(p-chlorophenoxy)-2-methylbenzyl-C-(tert-butylthio)imino]-pyrimidine (No. 2.15),
5-[4-(p-chlorophenoxy)-2-methylbenzyl-C-(isopropoxy)imino]pyrimidine (No. 2.16),
5-[4-(p-chlorophenoxy)-2-chlorobenzyl-C-(tert-butylthio)imino]pyrimidine (No. 2.12),
5-[4-(p-chlorophenoxy)-2-chlorobenzyl-C-(isopropoxy)imino]pyrimidine (No. 2.11),
5-[2,4-dichlorobenzyl-C-(isopropoxy)imino] pyrimidine (No. 1.12),
5-[2,4-dichlorobenzyl-C-(tert-butoxy)imino] pyrimidine (No. 1.13),
5-[2,4-dichlorobenzyl-C-(allyloxy)imino] pyrimidine (No. 1.88),
5-[2,4-dichlorobenzyl-C-(n-propoxy)imino] pyrimidine (No. 1.99),
5-[4-chlorobenzyl-C-(tert-butylthio)imino] pyrimidine (No. 1.17),
5-[2,4-dichlorobenzyl-C-(trifluoroethoxy)imino] pyrimidine (No. 1.14),
5-[2,4-dichlorobenzyl-C-(diethylamino)imino] pyrimidine (No. 1.95), and
5-[2,4-dichlorobenzyl-C-(tert-butylamino)imino] pyrimidine (No. 1.110).

16. A process for producing a compound of the formula I according to claim 1, which process comprises reacting a compound of the formula II

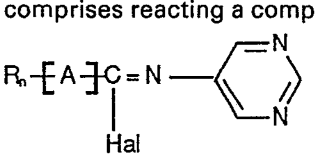 (II),

in the presence of a base, with a compound of the formula III

$R_1$-H (III),

the symbols $R_n$, A and $R_1$ in the formulae II and III having the meanings defined under the formula I, and Hal denoting halogen, preferably chlorine or bromine.

17. A microbicidal composition for controlling or preventing an infestation by microorganisms, which composition contains, as at least one active component, a compound of the formula according to claim 1.

18. A composition according to claim 17, which contains, as at least one active component, a compound of the formula I as claimed in claim 2.

19. A composition according to claim 17, which contains, as at least one active component, a compound of the formula I as claimed in claim 3.

20. A composition according to claim 17, which contains, as at least one active component, a compound of the formula I as claimed in any one of claims 4 to 15.

21. A process for controlling or preventing an infestation of cultivated plants by phytopathogenic microorganisms, which process comprises applying to the plants or to the locus thereof a microbicidally effective amount of a compound of the formula I.

22. A method of controlling plant diseases attributable to microorganisms, which method comprises applying a microbicidally effective amount of a compound of the formula I.

23. A method according to claim 22, which method comprises applying a compound of the formula I as claimed in any one of claims 2 to 15.

24. A method according to claim 22, wherein the microorganisms concerned are phytopathogenic fungi.

25. A compound of the formula II

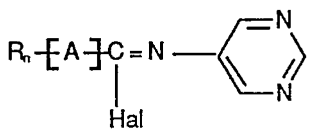 (II),

wherein $R_n$ and A have the meanings defined under the formula I in claim 1, and Hal is halogen, preferably chlorine or bromine.

## Revendications

1. Composés de formule I

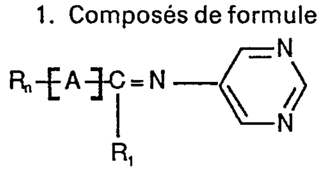 (I)

dans laquelle
A est le phényle, naphtyle, biphényle, phénoxyphényle ou phénylthiophényle; R est un halogène, cyano, nitro, alkyle $C_1$–$C_4$, alcoxy-carbonyle $C_1$–$C_4$, Tris(alcoxy $C_1$–$C_4$)silyl, hydroxy, alcoxy $C_1$–$C_4$, haloalcoxy $C_1$–$C_4$, haloalkyle $C_1$–$C_4$ ou di(alkyl $C_1$–$C_4$)amino; n est 0, 1, 2, 3, 4 ou 5; $R_1$ représente un des groupes $OR_2$, $SR_2$ ou $N(R_3)(R_4)$, dans lesquels $R_2$ est un alkyle $C_1$–$C_8$, alcényle $C_3$–$C_8$, alcynyle $C_3$–$C_8$ ou un reste alkyle $C_1$–$C_8$ substitué simplement par R, ou est un reste de la série phényle, phénalkyle ($C_1$–$C_4$), cycloalkyle $C_3$–$C_7$ ou furfuryle non substitué ou substitué par R de une à trois fois; et $R_3$ et $R_4$ indépendamment l'un de l'autre représentent l'hydrogène ou un alkyle $C_1$–$C_4$ ou forment ensemble avec l'azote de l'amine un hétérocycle saturé à cinq ou six membres, qui contient comme hétéroatome, soit seulement l'azote de l'amine, soit un autre hétéroatome N, O ou S.

2. Composés de formule I selon la revendication 1, dans laquelle A est le phényle, naphtyle, biphényle, phénoxyphényle ou phénylthiophényle; R est un halogène, cyano, nitro, alkyle $C_1$–$C_4$, alcoxy $C_1$–$C_4$, haloalcoxy $C_1$–$C_4$, haloalkyle $C_1$–$C_4$ ou di (alkyl $C_1$–$C_4$) amino; n est 0, 1, 2, 3, 4 ou 5; $R_1$ représente un des groupes $OR_2$, $SR_2$ ou $N(R_3)(R_4)$, dans lesquels $R_2$ est un alkyle $C_1$–$C_8$, alcényle $C_3$–$C_8$, alcynyle $C_3$–$C_8$, ou est un reste de la série phényle, benzyle, cycloalkyle $C_3$–$C_7$ ou furfuryle non substitué ou substitué par R de une à trois fois; et $R_3$ et $R_4$ indépendamment l'un de l'autre, représentent l'hydrogène ou un alkyle $C_1$–$C_4$ ou

forment ensemble avec l'azote de l'amine un hétérocycle saturé à cinq ou six membres, qui contient comme hétéroatome, soit seulement l'azote de l'amine, soit un autre hétéroatome.

3. Composés de formule I selon la revendication 1, dans laquelle A est le phényle; R est un hydroxy, halogène, cyano, méthyle, éthyle, méthoxycarbonyle, éthoxycarbonyle, éthoxy, $CF_3$, triméthoxysilyle, haloalcoxy $C_1$-$C_2$, $N(CH_3)_2$ ou $N(C_2H_5)_2$; n est 1, 2 ou 3; $R_1$ représente l'un des groupes $OR_2$ ou $SR_2$; $R_2$ représentant un alkyle $C_1$-$C_8$, alcényle $C_3$-$C_8$, alcynyle $C_3$-$C_8$ ou un reste alkyle $C_1$-$C_4$ simplement substitué par un hydroxy, halogène, cyano, méthoxycarbonyle, éthoxycarbonyle, méthoxy, éthoxy, triméthoxysilyle, triéthoxysilyle, $N(CH_3)_2$ ou $N(C_2H_5)_2$, ou est un reste de la série phényle, phénéthyle, benzyle, cycloalkyle $C_5$-$C_6$ et furfuryle non substitué ou substitué une à trois fois par un halogène, cyano, méthyle, éthyle, méthoxy, éthoxy, $CF_3$, haloalcoxy $C_1$-$C_2$ ou N(alkyl $C_1$-$C_2$)$_2$.

4. Composés de formule I selon la revendication 1, caractérisés en ce que A est le phényle; R est un halogène, cyano, méthyle, éthyle, méthoxy, éthoxy, $CF_3$, haloalcoxy $C_1$-$C_2$, $N(CH_3)_2$ ou $N(C_2H_5)_2$; n est 1, 2 ou 3; $R_1$ représente un des groupes $OR_2$ ou $SR_2$; dans lesquels $R_2$ est un alkyle $C_1$-$C_8$, alcényle $C_3$-$C_8$, alcynyle $C_3$-$C_8$ ou un reste de la série phényle, benzyle, cycloalkyle $C_5$-$C_6$ et furfuryle non substitué ou substitué une à trois fois par un halogène, cyano, méthyle, éthyle, méthoxy, éthoxy, $CF_3$, haloalcoxy $C_1$-$C_2$ ou N(alkyle $C_1$-$C_2$)$_2$.

5. Composés de formule I selon la revendication 4, caractérisés en ce que A est le phényle; R est le fluor, chlore, brome, méthyle, méthoxy ou $CF_3$; n est 1 ou 2; $R_1$ est $OR_2$ ou $SR_2$; dans lesquels $R_2$ est un alkyle $C_1$-$C_5$, alcényle $C_3$-$C_4$, alcynyle $C_3$-$C_4$, cyclopentyle, cyclohexyle, furfuryle ou un reste de la série phényle et benzyle non substitué ou substitué une à trois fois par le fluor, chlore, brome, méthyle, méthoxy ou $CF_3$.

6. Composé de formule I selon la revendication 5, caractérisés en ce que n est deux; R est le fluor ou le chlore; qui sont substitués en positions 2 et 4 du cycle phényle; $R_1$ est $OR_2$ ou $SR_2$; dans lesquels $R_2$ est un alkyle $C_1$-$C_5$, allyle, propargyle, cyclohexyle, benzyle ou un phényle non substitué ou substitué de une à trois fois par le fluor, chlore, brome, méthyle, méthoxy ou phényle substitué par $CF_3$.

7. Composés de formule I selon la revendication 1, dans lesquels A est le phénoxyphényle; n est 0, 1, 2, 3, 4 ou 5; et les autres substituants sont comme définis dans la revendication 3.

8. Composés de formule I selon la revendication 1, caractérisés en ce que A est le phénoxyphényle; R est un halogène, cyano, méthyle, éthyle, méthoxy, éthoxy, $CF_3$, haloalcoxy $C_1$-$C_2$, $N(CH_3)_2$ ou $N(C_2H_5)_2$, n est 0, 1, 2, 3, 4 ou 5; $R_1$ représente un des groupes $OR_2$ ou $SR_2$; $R_2$ étant un alkyle $C_1$-$C_8$, alcényle $C_3$-$C_8$, alcynyle $C_3$-$C_8$ ou un reste de la série phényle, benzyle, cycloalkyle $C_5$-$C_6$ ou furfuryle non substitué ou substitué une à trois fois par un halogène, cyano, méthyle, éthyle, méthoxy, éthoxy, $CF_3$, haloalcoxy $C_1$-$C_2$ ou N(aklyle $C_1$-$C_2$)$_2$.

9. Composés de formule I selon la revendication 8, caractérisés en ce que A est le (p-phénoxy)-phényle.

10. Composés de formule I selon la revendication 9, caractérisés en ce que A est le (p-phénoxy)phényle; R est le fluor, chlore, brome, méthyle, méthoxy ou $CF_3$; n est 0, 1, 2, 3 ou 4; $R_1$ est $OR_2$ ou $SR_2$; $R_2$ étant un alkyle $C_1$-$C_5$, alcényle $C_3$-$C_4$, alcynyle $C_3$-$C_4$, cyclopentyle, cyclohexyle, furfuryle ou un reste de la série phényle ou benzyle non substitué ou substitué de une à trois fois par le fluor, chlore, brome, méthyle, méthoxy ou $CF_3$.

11. Composés de formule I selon la revendication 10, caractérisés en ce que A représente le (p-phénoxy)-phényle; R est le fluor, chlore, méthyle, méthoxy ou $CF_3$; n est 0, 1, 2 ou 3; $R_1$ est $OR_2$ ou $SR_2$; $R_2$ étant un alkyle $C_1$-$C_5$, allyle, propargyle, cyclohexyle, benzyle ou un phényle non substitué ou substitué une à trois fois par le fluor, chlore, brome, méthyle, méthoxy ou $CF_3$.

12. Composés de formule I selon la revendication 1, dans laquelle A représente le phényle ou le p-phénoxyphényle; n est 0, 1, 2 ou 3, R est un halogène, cyano, méthyle, éthyle, méthoxy, éthoxy, méthoxycarbonyle, éthoxycarbonyle, $CF_3$ ou haloalcoxy $C_1$-$C_2$, $R_1$ est le groupe $N(R_3)(R_4)$ et $R_3$ et $R_4$ indépendamment l'un de l'autre, représentent l'hydrogène ou un alkyle $C_1$-$C_4$ ou forment ensemble avec l'azote de l'amine un cycle pyrrolidine, pipéridine, pipérazine ou morpholine.

13. Composés de formule I selon la revendication 12, dans laquelle R représente le fluor, chlore ou le méthyle.

14. Composés de formule I selon la revendication 1, dans laquelle A représente le p-biphényle, n = 0, $R_1$ est, soit $OR_2$, soit $SR_2$ et $R_2$ est un alkyle $C_1$-$C_4$, l'allyle ou le propargyle.

15. Composé de formule I selon la revendication 1, caractérisé en ce qu'il est choisi dans la série:

5-[2,4-dichlorobenzyl-C-(tert.butylthio)imino]pyrimidine (No 1.1.);
5-[4-(p-chlorophénoxy)-2-méthylbenzyl-C-(tert.butylthio)imino]pyrimidin (No 2.15);
5-[4-(p-chlorophénoxy)-2-méthylbenzyl-C-(isopropoxy)-imino]pyrimidine (No 2.16);
5-[4-(p-chlorophénoxy)-2-chlorobenzyl-C-(tert.butylthio)imino]pyrimidine (No 2.12);
5-[4-(p-chlorophénoxy)-2-chlorobenzyl-C-(isopropoxy)imino]pyrimidine (No 2.11);
5-[2,4-dichlorobenzyl-C-(isopropoxy)imino]pyrimidine (No 1.12);
5-[2.4-dichlorobenzyl-C-(tert.butoxy)imino]pyrimidine (No 1.13);
5-[2,4-dichlorobenzyl-C-(allyloxy)imino]pyrimidine (No 1.88);
5-[2,4-dichlorobenzyl-C-(n-propoxy)imino]pyrimidine (No 1.99);
5-[4-chlorobenzyl-C-(tert.butylthio)imino]pyrimidine (No 1.17);
5-[2,4-dichlorobenzyl-C-(trifluoréthoxy)imino]pyrimidine (No 1.14);

5-[2,4-dichlorobenzyl-C-(diéthylamino)imino]pyrimidine (No 1.95);
5-[2,4-dichlorobenzyl-C-(tert.butylamino)imino]pyrimidine (No 1.110).

16. Procédé de préparation de composés de formule I selon la revendication 1, caractérisé en ce qu'on fait réagir un composé de formule II

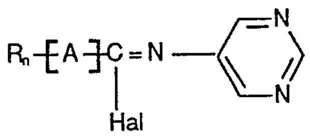 (II) ,

en présence d'une base avec un composé de formule III

$R_1$-H (III);

ici, $R_n$, A et $R_1$ dans les formules II et III, ont les significations indiquées pour la formule I, Hal représente un halogène, de préférence le chlore ou le brome.

17. Moyen de lutte contre les parasites pour combattre ou prévenir une attaque par des microorganismes, caractérisé en ce qu'il contient au moins comme composant actif un composé de formule I selon la revendication 1.

18. Moyen selon la revendication 17, caractérisé en ce qu'il contient au moins comme composant actif un composé de formule I selon la revendication 2.

19. Moyen selon la revendication 17, caractérisé en ce qu'il contient au moins comme composant actif un composé de formule I selon la revendication 3.

20. Moyen selon la revendication 17, caractérisé en ce qu'il contient au moins comme composant actif un composé de formule I selon l'une des revendications 4 à 15.

21. Procédé pour combattre ou prévenir une attaque des plantes cultivées par des microorganismes phytopathogènes, caractérisé en ce qu'on applique un composé de formule I sur les plantes ou leur terrain.

22. Utilisation de composés de formule I pour combattre des maladies des plantes, qui sont dues à des microorganismes.

23. Utilisation selon la revendication 22 de composés de formule I selon l'une des revendications 2 à 15.

24. Utilisation selon la revendication 22, caractérisée en ce qu'il s'agit de champignons phytopathogènes comme microorganismes.

25. Composés de formule II

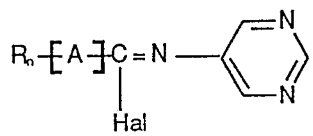 (II) ,

dans laquelle $R_n$ et A ont les significations données selon la revendication 1 et Hal est un halogène, de préférence le chlore ou le brome.